(19) Europäisches Patentamt
European Patent Office
Office européen des brevets

(11) Publication number: **0 392 745 B1**

(12) # EUROPEAN PATENT SPECIFICATION

(45) Date of publication of patent specification: **02.11.94**

(51) Int. Cl.[5]: **A61K 47/48**, A61K 37/54, C07D 501/00, C07D 499/00

(21) Application number: **90303681.2**

(22) Date of filing: **05.04.90**

(54) **Immunoconjugates and prodrugs and their use in association for drug delivery.**

(30) Priority: **05.04.89 GB 8907617**

(43) Date of publication of application:
**17.10.90 Bulletin 90/42**

(45) Publication of the grant of the patent:
**02.11.94 Bulletin 94/44**

(84) Designated Contracting States:
**AT BE CH DE DK ES FR GB GR IT LI LU NL SE**

(56) References cited:
**EP-A- 0 251 330**
**EP-A- 0 302 473**
**EP-A- 0 335 297**
**EP-A- 0 382 411**
**FR-A- 2 501 209**

**BR. J. CANCER,vol. 58, 1988, pages 700-703; K.D.BAGSHAWE et al.: "A cytotoxic agent can be generated selectively at cancer sites"**

(73) Proprietor: **CELLTECH LIMITED**
**216 Bath Road**
**Slough Berkshire SL1 4EN (GB)**

(72) Inventor: **Eaton, Michael Anthony William**
**Nethercote,**
**Chinnor Road**
**Aston Rowant, Oxfordshire OX9 5SH (GB)**
Inventor: **Pratt, Andrew John**
**Jesus College**
**Oxford OX1 3DW (GB)**
Inventor: **Alexander, Rikki Peter**
**2 Warwick Avenue**
**High Wycombe, Buckinghamshire PH12 4NG (GB)**

(74) Representative: **Hallybone, Huw George et al**
**CARPMAELS AND RANSFORD**
**43 Bloomsbury Square**
**London WC1A 2RA (GB)**

**EP 0 392 745 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

STN FILE/ FILE CA, Chemical Abstract, vol. 111, 1989, abstract no.49976x, R.N.JONES et al.: "In vitro activity of Ro 23-9424, ceftazidime, and eight other newer beta-lactams against 100 gram-positive blood culture isolates", & DIAGN. MICROBIOL. INFECT. DIS., 12(2), 143-7.

J. MED. CHEM., vol. 33,1990,pages 77-86, American Chemical Society; H.A.ALBRECHT et al .. "Cephalosporin 3 - quinolone esters with a duel mode of action"

## Description

### Field of the Invention

This invention relates to a system for the targetted delivery of drugs to humans. More particularly, it relates to immunoconjugates, to prodrugs, and to their use in association for the targetted delivery of drugs to humans.

### Background to the Invention

There are numerous drugs available, for example for the treatment of cancer, which can not be effectively utilised by standard systemic administration because of toxic effects to the normal tissues of the host. In such instances, the success of any treatment with the drug will depend to a large extent on the ability to selectively target it to the diseased tissue. Unfortunately, many previous attempts to target drugs specifically have failed because of the lack of qualitative and quantitative biochemical differences between normal and diseased tissue.

The advent of monoclonal antibody technology, however, has provided a means for selective targetting of tissues. Thus, by coupling or complexing an antibody with a drug in such a way that the resulting immunoconjugate retains the antigenic binding properties of the antibody and activity of the drug, it is possible to direct and convey the drup to a selected tissue.

The success of this approach depends on achieving an effectively high concentration of the drug containing immunoconjugate at the disease site and maintaining this concentration for a sufficient length of time for the drug to exert the desired therapeutic effect. In practice this has proved to be a significant problem, since in most cases reported to date very little of the immunoconjugate dose is found to localise at the desired site, resulting in relatively low levels of delivery of the drug.

It is undesirable from both a clinical and economic point of view to offset this difficulty by increasing the dose of immunoconjugate. Increasing dosage may increase side effects in the patient such as immune response and non specific toxicity. A requirement for large amounts of immunoconjugate is also undesirable because of its high cost of production. Similarly, increasing the toxicity of the drug attached to the antibody to offset this difficulty is also likely to increase toxic side effects on normal tissue. There has therefore been a need to improve monoclonal antibody based drug targetting methods such that the delivered dose of drug is increased without radical increase in the applied dose of immunoconjugate.

One recently suggested method for overcoming this problem employs a monoclonal antibody-enzyme-prodrug system, described more particularly in European Patent Applications EP-A-0187658 and EP-A-0302473 and International Patent Application WO88/07378
In a particular example of the method, a monoclonal antibody or antibody fragment specific for a tumour associated antigen [i.e. an antigen present in relatively high levels on the surface of or within tumour cells compared with normal cells where it may also be present] is linked to an enzyme using chemical cross linking, or recombinant DNA gene fusion and expression techniques. The linkage is achieved in such a manner that the resulting immunoconjugate retains both the antigen recognition ability of the antibody and the catalytic activity of the enzyme. A substrate for the enzyme is synthesised which consists of a tumouricidal drug in an innocuous non-tumouricidal form (termed a prodrug). The prodrug is converted into the tumouricidal drug form by the action of the enzyme.

In order to achieve an antitumour effect the immunoconjugate is first injected or infused into the patient and allowed to localise at the site of the tumour. A period of time is allowed to elapse in which non-tumour bound immunoconjugate is allowed to largely clear from the patient by metabolism and excretion. The prodrug is then injected or infused. Upon reaching the tumour, the prodrug is converted to drug by the enzyme portion of the bound immunoconjugate. The dose of prodrug given will be that required to generate a therapeutically effective concentration of drug at the site of the tumour. The de novo created drug then exerts its tumouricidal effect in the immediate vicinity of the tumour thus tending to minimise exposure of normal tissue to its effects. The clearance period after infusion of the immunoconjugate is aimed at minimising the level of immunoconjugate in non-tumour tissue. This is required to limit generation of drug from prodrug by the enzyme portion of the immunoconjugate in non-tumour tissue. Treatment is halted by the termination of infusion of the prodrug.

The method thus rests on an enzyme-mediated drug amplification effect in which the number of toxic molecules is greatly increased over the number of immunoconjugate molecules at the site of the tumour. This achieves an effective local increase in concentration of the drug without the need to resort to an increase in dose of the immunoconjugate.

Particular antibody-enzyme-prodrug systems of the above type which have been described include those in which urokinase, (European Patent Application EP-A-0187658 carboxypeptidase $G_2$, [International Patent Application WO88/07378; Bagshawe et al, Br. J. Cancer 58, 700 - 703, (1988)] or alkaline phosphatase, penicillin V amidase and cytosine deaminase, [European Patent Application EP-A-0302473; Senter et al PNAS (1988), 85, 4842-4846] have been coupled to antibodies for use with plasminogen, benzoic acid mustard, etoposide phosphate, N-(p-hydroxyphenoxyacetyl)adriamycin or 5-fluorocytosine prodrugs, respectively.

The earlier application EP-A-0382411, which was published after the priority date of the present invention, discloses systems for the treatment of neoplastic disease. The systems comprise an immunoconjugate having β-lactamase activity and a prodrug which is a cephalosporin having a cytotoxic agent linked thereto at the 3 position. The immunoconjugate comprises an antibody or antibody fragment conjugated to a β-lactamase enzyme by means of a chemical conjugating agent such as sulfo-SMCC. The antibody part of the immunoconjugate is capable of recognising and binding to neoplastic cells, where the β-lactamase part of the immunoconjugate catalyses the decomposition of the cephalosporin prodrug with release of the cytotoxic agent.

The present invention concerns an antibody-enzyme-prodrug delivery system of the above general type, wherein active drug is generated from inactive prodrug at the host target site by the action of a β-lactamase enzyme conveyed there by an appropriate antibody. European Patent Application EP-A-0302473 mentions the possibility of using inter alia antibody-β-lactamase conjugates with, generally, drugs derivatised with β-lactams.

It will be appreciated that β-lactamases are capable of hydrolysing a wide range of β-lactam substrates. In certain substrates, hydrolysis is also accompanied by elimination of a leaving group. In the present invention, we have designed substrates for β-lactamases in which use is made of this mechanism to provide a means for generating active drug from inactive drug.

Thus according to one aspect of the invention we provide a product for delivering a drug at a host target site, the product comprising an immunoconjugate and a prodrug as a combined preparation for sequential use in therapy, said immunoconjugate being capable of recognizing and binding to one or more epitopes associated with the host target site and having a β-lactamase action capable of hydrolysing said prodrug to active drug or an unstable precursor thereof at the target site, characterised in that said immunoconjugate comprises a β-lactamase enzyme or fragment thereof directly linked to an antibody or antibody fragment capable of recognizing and binding to the said one or more epitopes, and the prodrug is a cephalosporin or penicillin derivative of formula (3)

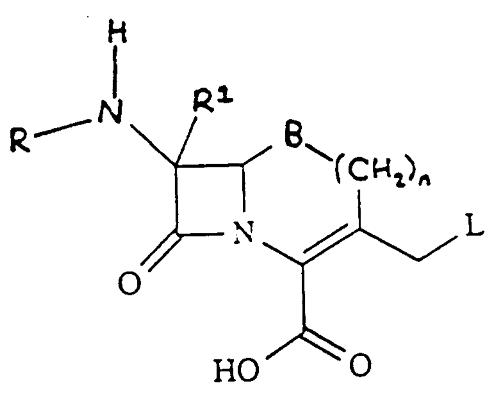

(3)

wherein R is an acyl or alkyl radical: $R^1$ is a hydrogen atom or an alkoxy group; B is $-CH_2-$, -O-, or -S-, n is zero or an integer 1 to 4 inclusive, L is the active drug or unstable precursor thereof and L is linked to the remainder of the molecule such that it forms a leaving group, whereby L is eliminated on hydrolysis of the prodrug.

The term epitope as used herein is intended to mean any immunogenic site to which an antibody may recognise and bind.

The system according to the invention may be used for delivering a drug at any host target site where treatment is required, providing the target site has one or more epitopes that are substantially unique to that site, and which can be recognised and bound by the immunoconjugate. Particular target sites include those regions in a host arising from a pathogenic state induced by, for example a tumour, a bacterium, a fungus

or a virus; or as a result of a malfunction of a normal host system, for example in cardiovascular diseases, such as the formation of a thrombus, in inflammatory diseases, and in diseases of the central nervous system.

In the delivery system according to the invention, the immunoconjugate may in general comprise at least an antigen binding domain of an antibody and at least the active portion of a $\beta$-lactamase directly linked such that each is separately functional. Thus, the immunoconjugate may be a whole antibody or an antigen binding fragment thereof, directly linked to a $\beta$-lactamase enzyme or an active fragment thereof.

The antibody or antibody fragment may in general belong to any immunoglobulin class. Thus, for example, it may be an immunoglobulin M antibody or, in particular, an immunoglobulin G antibody.

Particular recombinant antibodies or antibody fragments include, (1) those having an antigen binding site at least part of which is derived from a different antibody, for example those in which the hypervariable or complementarity determining regions of one antibody have been grafted into the variable framework regions of a second, different antibody (as described in European Patent Specification EP-A-0239400); (2) recombinant antibodies or fragments wherein non-Fv sequences have been substituted by non-Fv sequences from other, different antibodies (as described in European Patent Specifications EP-A-0171496, EP-A-0173494 and EP-A-0194276); or (3) recombinant antibodies or fragments possessing substantially the structure of a natural immunoglobulin but wherein the hinge region has a different number of cysteine residues from that found in the natural immunoglobulin, or wherein one or more cysteine residues in a surface pocket of the recombinant antibody or fragment is in the place of another amino acid residue present in the natural immunoglobulin, or wherein one or more cysteine residues in a surface pocket of the recombinant antibody or fragment is in the place of another amino acid residue present in the natural immunoglobulin.

The antibody or antibody fragment may be of polyclonal, for, preferably, monoclonal origin. It may be specific for a number of epitopes associated with the host target site, but is preferably specific for one.

Antigen binding antibody fragments include fragments obtained by recombinant DNA techniques, for example Fv fragments (as described in International Patent Application W089/02465). Other fragments include peptides related to the so-called complementarity determining region of antibodies which may possess the ability to bind antigen.

The $\beta$-lactamase to which the antibody or antibody fragment is linked to form the immunoconjugate may in general be a $\beta$-lactamase (EC 3.5.2.6) from any prokaryotic source. Typical sources include Eschericia, Staphylococci, Pseudomonas, Bacteriodes, Klebsiella, Citrobacter, Bacillus, Enterobacter, and Streptococci. Particular cloacae and E. coli, especially E. coli R-TEM. Fragments of $\beta$-lactamases may also be used, for example fragments produced by expression of a truncated or modified form produced by recombinant DNA technology, providing enzyme activity is retained.

The $\beta$-lactamase or fragment thereof is linked to the antibody or antibody fragment directly to form the immunoconjugate for use in the invention. Direct linkage is to be understood to mean peptide bond formation between the C-terminal amino acid of a heavy or light chain of the antibody or fragment and the N-terminal amino acid of the $\beta$-lactamaseor fragment thereof.

Prodrugs for use in the delivery system according to the invention are cephalosporin and penicillin derivatives of formula (3):

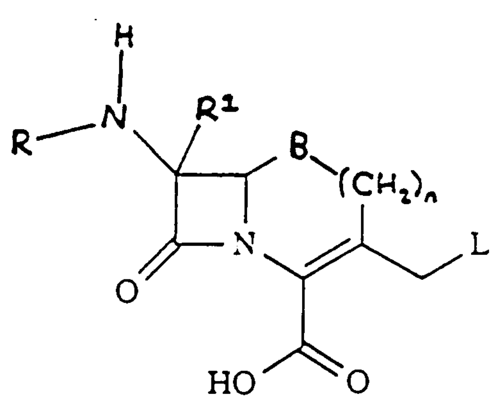

(3)

(wherein R is an acyl or alkyl radical; $R^1$ is a hydrogen atom or an alkoxy group; B is $-CH_2-$, -O-, or -S- and n is zero or an integer 1 to 4 inclusive; and L is a drug or unstable precursor thereof as defined in claim 8 linked to the remainder of the molecule such that it forms a leaving group.

In the compounds of formula (3) the acyl group represented by the group R may be for example any acyl group known from the penicillin and cephalosporin art. Thus R may be for example an optionally substituted aliphatic, heteroaliphatic, aromatic, heteroaromatic, araliphatic, or heteroaliphatic carboxylic or carbothioic acid radical or a carbamoyl radical.

One particular group of acyl groups represented by R are those of formula $R^2C=X$, where X is an oxygen or sulphur atom and $R^2$ represents a hydrogen atom or a group selected from amino, substituted amino e.g. $-NR^3R^4$ (where $R^3$ and $R^4$ which may be the same or different is each a hydrogen atom or a $C_{1-6}$ alkyl group), $C_{1-6}$ alkyl, $C_{1-6}$ alkylthio, $C_{6-12}$ arylthio, $C_{1-6}$ alkoxy, $C_{2-6}$alkenyl or alkynyl, aryl, e.g. phenyl, ar$C_{1-3}$alkyl, e.g. benzyl, $C_{3-6}$ cycloalkyl, $C_{4-10}$ heteroaryl or heteroar$C_{1-3}$alkyl where the heteroatom or atoms are selected from O, N or S, e.g. thienyl or thienylmethyl. Each of the above groups may be optionally substituted by further atoms or groups, for example by halogen atoms, e.g. chlorine atoms, or by groups such as -OH, $-SR^5$ (where $R^5$ is a hydrogen atom or an alkyl or aryl group), $C_{1-6}$alkyl, $C_{1-6}$alkoxy, cyano, carboxy, sulphamino, carbamoyl, suphonyl, azido, amino, substituted amino (as defined above) halo$C_{1-6}$ alkyl, e.g. trifluoromethyl, carboxy$C_{1-6}$alkyl carbamoyl$C_{1-6}$alkyl, N-carbamoyl$C_{1-6}$alkyl, amidino, guanidino and substituted guanidino.

When R is an alkyl group it may be for example a straight or branched $C_{1-6}$alkyl group, e.g. methyl, ethyl, n-propyl, n-pentyl or n-hexyl group.

It will be appreciated that the group R may be varied widely without affecting the usefulness of the delivery system according to the invention. One group R however which we have found to be particularly useful is 2-thienylacetyl.

The group $R^1$ in the above compounds may be a $C_{1-6}$alkoxy e.g. methoxy group, but is preferably a hydrogen atom.

In the above compounds, β-lactamase hydrolysis of the cyclic amide also results in liberation of the drug or unstable precursor, providing either is linked to the remainder of the molecule in such a manner that it forms a leaving group. Thus, the linkage will generally be through an oxygen, nitrogen or sulphur atom present in the drug. Particular examples of L include $-O-CO-L^1$, and $-S-L^1$ where $L^1$ is the remainder of the drug or unstable precursor. Preferred linkages are those that are not susceptible to cleavage by host enzyme mechanisms (e.g. esters may be cleaved by host esterases), to avoid premature release of the drug at sites other that the target site.

The term unstable precursor as used herein in relation to a drug is intended to mean a metabolically or inherently unstable precursor of the drug. Metabolically unstable precursors of the drug are those from which active drug may be generated by host cell mechanisms at the host target site. Inherently unstable precursors of the drug are those which spontaneously decompose to active drug at the host target site. Particular examples include carbonate, thiocarbonate, carbamate and thiocarbamate derivatives of the drug.

The term drug as used herein is intended to mean any physiologically active substance, antibacterial, antiviral or antifungal compound. Particular physioligically active substances include antineoplastic agents, including cytotoxic and cytostatic agents, hormones, anti-inflammatory compounds, and substances active as cardiovascular, e.g. fibrinolytic, and central nervous system agents.

The delivery system according to the invention is particularly useful for targetting drugs to tumours and in a preferred aspect the invention thus provides a system for delivering a drug to a tumour, the system comprising an immunoconjugate and a prodrug for use in association with each other, said immunoconjugate being capable of recognising and binding to one or more tumour associated epitopes and having a β-lactamase action capable of hydrolysing said prodrug to active drug or an unstable precursor thereof at the tumour site, characterised in that said prodrug comprises a cyclic amide derivative of a drug or an unstable precursor thereof wherein the drug or an unstable precursor thereof is linked to the remainder of the such that it forms a leaving group which on hydrolysis of the prodrug is eliminated as the active drug or an unstable precursor thereof.

According to this aspect of the invention, the immunoconjugate may generally be as described previously, providing it is capable of binding to at least one tumour associated epitope. Particular epitopes include oncofetal antigens such as carcinoembryonic antigen or alphafetoprotein, placental antigens such as chorionic gonadotropin and placental alkaline phosphatase, and prostate antigens such as prostatic acid phosphatase and prostate specific antigen.

Immunoconjugates capable of recognising and binding one or more epitopes on the TAG-72 antigen associated with human breast and colon tumours are particularly useful in delivery systems according to this aspect of the invention. Particularly preferred immunconjugates of this type are those wherein the monoclonal antibody B72.3 [Colcher, D. et al Proc. Nat. Acad. Sci. USA (1981), 78, 3199] or a fragment thereof or a recombinant B72.3 antibody or fragment thereof is directly linked to a β-lactamase. Recombinant B72.3 antibodies or fragments include those of the type described above in relation to antibodies

generally.

Suitable prodrugs for use in this aspect of the invention include inactive forms of antineoplastic drugs from which the active form or an unstable precursor may be generated by the action of a $\beta$-lactamase.

Particular antineoplastic agents include cytotoxic and cytostatic agents, for example alkylating agents, such as nitrogen mustards (e.g. chlorambucil, melphalan, mechlorethamine, cyclophosphamide, or uracil mustart) and derivatives thereof, triethylenephosphoramide, triethylenethiophosphoramide, busulphan, or cisplatin; antimetabolites, such as methotrexate, fluorouracil and floxuridine, cytarabine, mercaptopurine, thioguanine, fluoroacetic acid or fluorocitric acid; antibiotics, such as bleomycins (e.g. bleomycin sulphate), doxorubicin, daunorubicin, mitomycins (e.g. mitomycin C), antinomycins (e.g. dactinomycin) plicamycin, calicheamicin, or esperamicin; mitotic inhibitors, such as etoposide, vincristine or vinblastine and derivatives thereof; alkaloids, such as ellipticine; polyols such as taxixin-I or taxicin-II; hormones, such as androgens (e.g. dromostanolone or testolactone), progestins (e.g. megestrol acetate or medroxyprogesterone acetate), estrogens (e.g diethylstilbestrol diphosphate, polyestradiol phosphate or estramustine phosphate) or antiestrogens (e.g. tamoxifen); ureas, such as hydroxyurea; hydrazines, such as procarbazine; or imidazoles, such as dacarbazine.

Particularly useful prodrugs for use in delivery systems according to this aspect of the invention are those of formula (3) wherein R, $R^1$, B and n are as defined for formula (3) and L is a group $-OCOL^1$ where $L^1$ is a group $-NR^6R^7$ wherein $R^6$ and $R^7$ which may be the same or different is each a hydrogen atom or an optionally substituted $C_{1-6}$alkyl [e.g. ethyl or propyl] or nitroso group, with the proviso that only one of $R^6$ or $R^7$ is a hydrogen atom; or a group $-P-NR^6R^7$ where P is a phenyl group. Examples of the substituents which may be present on $R^6$ and $R^7$ alkyl groups include halogen atoms, e.g. chlorine or bromine atoms.

Another important group of compounds of formula (3) are those wherein L is a mercaptopurine or thioguanine group.

Compounds of formula (3) in which L is an antineoplastic agent as defined above, R and $R^1$ are as defined for formula (3), especially where $R^1$ is a hydrogen atom, B is -S- and n is an integer 1 are particularly important. Compounds of this type in which L is a group $-OCOL^1$ as just defined or a mercaptopurine or thioguanine group are especially useful.

Compounds of the above specific types form a further feature of the invention.

The efficacy of a system according to the invention may be initially determined using appropriate model in vitro or in vivo test systems, for example by use of in vitro cell killing systems as described by Phillips (1974), Biochem. Pharmacol, 23, 131-138 or a mouse xenograft model as described by Searle et al (1981) in Br.J.Cancer 44, 137-144, and Bagshawe et al (1988) in Br.J.Cancer 58, 700-703.

The system according to the invention may be used in humans as generally described previously. Thus, the immunoconjugate will be administered first, and a period of time then allowed to elapse before the prodrug is administered. The period of time between the end of administration of the immunoconjugate and the beginning of administration of prodrug will vary depending on the site to be targetted and the nature of the immunoconjugate and prodrug, together with other factors such as the age and condition of the patient. Thus the exact regime will usually need to be determined empirically, with the aim of achieving a maximal concentration of immunoconjugate at the target site and a minimal concentration elsewhere in the patient, before the prodrug is administered. In this way, an optimum selective therapeutic effect can be achieved.

In practice, it may be desirable to space apart administration of immunoconjugate and prodrug by at least 4 hours. More than one administration of prodrug may be necessary to achieve the desired therapeutic effect.

The immunoconjugate and prodrug may be administered by any suitable route, preferably parenterally, e.g. by injection or infusion. Suitable formulations of the immunoconjugate or prodrug for parenteral administration include suspensions, solutions or emulsions of each component in oily or aqueous vehicles, and may contain formulatory agents such as suspending, stabilising and/or dispersing agents. Alternatively, the immunoconjugate or prodrug may be in powder form for reconstitution with a suitable vehicle. e.g. sterile pyrogen-free water, before use. If desired, the immunoconjugate and/or prodrug may be presented in unit dosage form.

The precise doses at which the immunoconjugate and prodrug will be administered will depend on the route of administration, body weight and pathology of the patient, the nature of the prodrug, and the catalytic properties of the $\beta$-lactamase. Thus, for example, the immunoconjugate may be administered at doses in the range 100 to 2000 U/kg. The prodrug may be adminstered at doses in general use for the administration of the drug itself, but will preferably be administered at lower doses, for example, of around 0.001 to 0.5 times the normally administered dose of drug alone.

Immunoconjugates for use in the delivery system according to the invention may be prepared by linking an antibody or antibody fragment to a β-lactamase or a fragment thereof by recombinant DNA technology.

Standard recombinant DNA techniques may be used. Thus, the DNA encoding the heavy (H) chain of an antibody (or a fragment of the H chain bearing the antigen combining site) may be ligated to the DNA sequence encoding the β-lactamase or fragment thereof. The resulting H chain-enzyme hybrid may then be expressed and secreted from transformed mammalian or microbial cells in culture together with the appropriate light (L) chain (or L chain fragment). The expressed antibody H chain-enzyme hybrid associates with the expressed L chain to form a functional Fv, Fab or full length HL antibody half molecule depending on the portion of the H chain chosen for expression. Conversion of the resulting monomeric HL species into dimers via cross linking at sulphydryl residues located in the hinge antibody region if present may occur spontaneously by air oxidation during cell culture, or may be achieved using appropriate oxidising agents or by reaction with chemical cross-linkers.

Alternatively, the DNA of the light chain of the antibody (or fragment) may be ligated to the β-lactamase or a fragment thereof. The resulting hybrid may be expressed with the appropriate H chain as described above.

Antibodies or antibody fragments may be obtained by conventional means, for example from the sera of immunised animals, or, preferably, myeloma or hybridoma cells, or by recombinant DNA techniques as described in European Patent Specifications EP-A-0171496, EP-A-0173494, EP-A-0194276 and EP-A-0239400.

β-Lactamases for use in the immunoconjugates are either widely available or may be obtained from known sources using standard techniques. The degree of suitability of any particular β-lactamase for use in the invention may be determined before or after conjugation to antibody using small scale screening tests, for example by determining the in vitro hydrolysis of a prodrug by the enzyme as described in more detail below.

Prodrugs for use in the delivery system according to the invention may be prepared by reaction of the drug or a metabolically unstable precursor thereof with a suitably activated cyclic amide derivative. In the following description reference is made to the preparation of compounds of formula (3).

Thus, a compound of formula (3) may be prepared by reaction of a compound of formula (5):

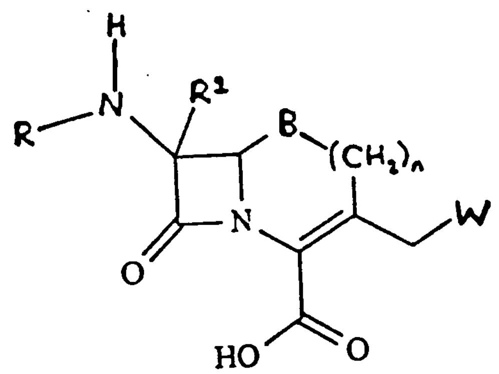

(5)

[wherein W is a reactive group such as a -CHO, -OH, $-OR^a$(where $R^a$ is a sulphonyl group such as a methanesulphonyl or p-toluenesulphonyl group), Hal (where Hal is a halogen atom such as a chlorine or iodine atom), $-NH_2$, $-OCONH_2$ or $-OCOCH_3$ group] with a drug or metabolically unstable precursor thereof under conditions such that either nucleophilic displacement of, or electrophilic addition to, the group W is achieved via a suitable reactive group [for example a thiol, isocyanate, carboxyl, activated carboxyl, or hydroxyl group] in the drug or precursor. The reaction may be effected in an aqueous or organic solvent, for example a halogenated hydrocarbon, e.g. dichloromethane or a subsituted amide, e.g. dimethylformamide using standard conditions for example in the presence of a base such as pyridine.

Alternatively, if desired, a compound of formula (3) may be prepared from a compound of formula (5) in a multi-stage reaction wherein the drug or an unstable precursor thereof is synthesised in step-wise fashion on the cyclic amide nucleus. Thus, for example, a compound of formula (5) wherein W is a hydroxyl group may be reacted with an acid ($R^8COOH$) or an activated derivative thereof (e.g. an acid halide or anhydride) to yield an intermediate compound wherein W is a group $-OCOR^8$. The reaction may be performed under standard conditions, for example in an aqueous or organic solvent, for example an ether such as tetrahydrofuran, where necessary in the presence of an acid or base, e.g. an organic amine such as

pyridine. By appropriate initial choice of the group $R^8$ such that it forms a good leaving group (e.g. a p-nitrophenoxy or pentafluorophenoxy group) the intermediate may be subsequently reacted with a drug or a precursor thereof (for example as described above) to yield the desired compound of formula (3).

Where a precursor of the drug is used this may be subsequently converted to the drug using standard reactions and conditions, for example as described in the following Examples. Thus for example where the drug contains a halogen atom this may be introduced in a final step by reaction of a corresponding compound containing a hydroxyl group with a sulphonyl chloride (e.g. methanesulphonyl chloride) in a base such as pyridine followed by reaction with a N-haloimide e.g. N-chlorosuccinimide. Alternatively chlorine and bromine atoms may be interconverted in a final step for example by treatment of a chlorine containing drug with a bromophosphorane e.g. dibromotriphenylphosphorane in the presence of a base such a pyridine. In another example, N-nitroso groups may be introduced in a final step by reaction of a corresponding secondary amine with $N_2O_4$.

It will be appreciated that in reactions of the above kind, it may be desirable to protect other reactive groups, for example carboxylic acid groups, in the cyclic amide or drug to avoid the possibility of side reactions occurring. Conventional protection procedures may be used, (for example carboxylic acids may be protected as esters e.g. diphenylmethyl esters) such that the protecting group may be conveniently removed without affecting the remainder of the molecule once the desired reaction has been effected.

In the above synthetic procedures we have found that compounds of formula (5) wherein W is a group -OCOOW$^1$ where W$^1$ is a fluorophenyl group, especially a pentafluorophenyl group, are particularly useful intermediates. Such compounds are new and form a further aspect of the invention.

Where the intermediate compounds of formula (5) are cephalosporin or penicillin derivative, these may be obtained by conventional methods from known starting materials, for example as described by C. F. Murphy and J. A. Webber, in "Cephalosporins and Penicillins: Chemistry and Biology", ed. E. H. Flynn, pp 134-182, Academic Press 1972. Other cyclic amides of formula (5) together with starting materials for use in the preparation of compounds of formula (3) may be prepared from known starting materials using analogous methods to those used for the preparation of the cephalosporin derivatives.

The following Intermediates and Examples illustrate the preparation of prodrugs and antibody-β-lactamase conjugates for use according to the invention.

## Description of Specific Embodiments

### Intermediate 1

### 3-Hydroxymethyl-7β-(2-thienylacetamido)-3-cephem-4-carboxylic Acid

To a solution of cephalothin sodium salt (12.2g) in water (60ml) at 0°C was added in one portion, 20% NaOH solution (120ml) previously cooled in a dry ice/acetone bath to -20°C. The temperature immediately increased to -4°C and was then reduced to -10°C. The mixture was agitated by hand for 135s, including cooling period. Upon rapidly quenching the reaction with glacial acetic acid (39ml) the temperature increased to +15°C. After recooling to +10°C, concentrated hydrochloric acid (70ml), precooled to 0°C, was added until the acid just precipitated (pH = 1.5). The resulting acid was washed twice with cold water and dried in vacuo overnight, to yield the title compound (7.4g)

m.p.: 214-215°C (dec.); I.R. $\nu_{max.}$ (Nujol): 3500 (alcohol), 3280, 2900, 1760 (β-lactam), 1715, 1640 (amide) $cm^{-1}$; $^1$H nmr $\delta_H$ ($D_2O$, $NaHCO_3$): 3.25 and 3.45 (2H, ABq, J 17.8 Hz, C-2$H_2$) 3.70 and 3.77 (2H, ABq, J 15.8 Hz, C-7$CH_2$), 4.06 and 4.11 (2H, ABq, J 12.9 Hz, C-3$CH_2$), 4.93 (1H, d, J 4.7 Hz, C-6H), 5.44 (1H, d, J 4.7 Hz, C-7H), 6.87-6.89 (2H, m, 2 thiophene H) and 7.19-7.21 (1H, m, thiophene H).

### Intermediate 2

### Diphenylmethyl 3-Hydroxymethyl-7β-(2-thienylacetamido)-3-cephem-4-carboxylate

To deacetyl cephalothin (14.8g) in acetone (150ml) was added a solution of diphenyl diazomethane (9.7g) in acetone (100ml). The mixture was stirred at room temperature under nitrogen for 40 min. The product was precipitated with hexane (150ml), filtered, washed with hexane, and dried in vacuo, to yield the title compound (13.1g), I.R. $\nu_{max.}$ ($CHCl_3$): 3400, 3010, 1790 (β-lactam), 1710 (ester), 1685 (amide) $cm^{-1}$; $^1$H nmr$\delta_H$ ($CDCl_3$): 3.55 (2H, s, C-7$CH_2$), 3,94 and 4.40 (2H, ABq, J 12.9 Hz, C-2$H_2$), 4.94 (1H, d, J 4.9 Hz, C-6H), 5.90 (1H, dd, J 4.9 and 9.2 Hz, C-7H), 6.44 (1H, d, J 9.2 Hz, C-7NH), 6.91 (1H, s, C-4CH), 6.97-7.02 (2H, m, 2 thiophene) and 6.97-7.02 (11H, m, 11 thiophene and phenyl H); MS (FD): m/z 520 ($M^+$)

Intermediate 3

Diphenylmethyl 3-Pentafluorophenoxycarbonoylmethyl-7$\beta$-(2-thienylacetamide)-3-cephem-4-carboxylate.

To a stirred solution of phosgene (2.07 ml of a 1.93M solution) in dry tetrahydrofuran (THF) (10 ml) at 0°C was added pyridine (0.323ml) followed by pentafluoro phenol (0.736g) as a solution in THF (4ml). The mixture was stirred at 0°C for 30 minutes and then Intermediate 2 (2.08g) and pyridine (0.323ml) added together as a solution in THF (5ml) dropwise. The reaction mixture was stirred at 0°C for 30 minutes and then at room temperature for 1 hour then poured into saturated $NaHCO_3$ solution (150ml) and extracted twice with $CH_2Cl_2$ (150ml). The combined organic fractions were dried over $MgSO_4$, filtered and the solvent removed by evaporation in vacuo to yield the title compound (2.61g) I.R. $\nu_{max.}$ (KBr): 2970, 1778 ($\beta$-lactam), 1726 (ester), 1692 (carbonate), 1680 (amide) $cm^{-1}$; $^1$H nmr$\delta_H$ ($CDCl_3$): 3.42 and 3.61 (2H, ABq, J 18.75 Hz, C-$2H_2$), 3.85 (2H, s, C-$7CH_2$), 5.01 and 5.35 (2H, ABq, J 15 Hz, C-$3CH_2$), 5.0 (1H, d, J 6 Hz, C-6H), 5.90 (1H, dd, J 6 and 8.25 Hz, C-7H), 6.74 (1H, d, j 8.25 Hz, C-7NH), 6.88 (1H, s, C-4CH), 6.90-7.00 (2H, m, 2H thiophene), and 7.18-7.25 (13H, m, 11 thiophene and phenyl, H).

Intermediate 4

Diphenylmethyl 3-(bis-2-hydroxyethyl carbamoyl)-7$\beta$-(2-acetamido)-3-cephem-4-carboxylate

To a stirred solution of Intermediate 3 in dry pyridine (10ml) at 0°C was added diethanolamine (0.32g) as a solution in pyridine (5ml). The mixture was stirred for 2 hrs. The solvent was removed by evaporation in vacuo, the residue dissolved in $CH_2Cl_2$ (100ml) and washed with saturated $NaHCO_3$ solution (100ml). The organic layer was dried over $MgSO_4$, filtered and the solvent removed by evaporation in vacuo. Purification was by column chromatography eluting with ethyl acetate to yield the title compound (0.3g) $^1$H n.m.r.$\delta_H$ - ($CDCl_3$): 3.32-3.51 (6H, m, C-$2H_2$ and C-$3NCH_2$) 3.59-3.82 (4H, m, C-$3CH_2OH$), 3.83 (2H, s, C-7CH), 4.87 and 5.03 (2H, ABq, J 15 Hz, C-$3CH_2$), 5.91 (1H, d, J 6 Hz, C-6H), 5.88 (1H, dd, J 6 and 9 Hz, C-7H), 6.93 (1H, s, C-4CH), 6.95-7.02 (3H, m, 2 thiophene and C-7NH) and 7.18-7.45 (11H, m, 10 phenyl and 1 thiophene H); MS (+ve FAB): m/z 652 (M+1).

Intermediate 5

Diphenylmethyl 3-(bis-2-methanesulphonylethylcarbamoyl)-7$\beta$-(2-acetamido) -3-cephem-4-carboxylate

To a stirred solution of Intermediate 4 (0.19g) in pyridine (5ml) was added methanesulphonyl chloride (0.133g) dropwise. The mixture was stirred overnight at room temperature and then poured into saturated $NaHCO_3$ solution (100ml) and extracted twice with $CH_2Cl_2$ (100ml). The combined organic fractions were dried over $MgSO_4$, filtered and the solvent removed by evaporation in vacuo. The product was purified by column chromotography eluting with 25% ethyl acetate/$CH_2Cl_2$, to yield the title compound (0.076g), $^1$H nmr$\delta_H$ ($CDCl_3$): 2.95 (6H, s, $OSO_2CH_3$), 3.34-3.66 (6H, m, C-$2H_2$ and C-$3NCH_2$), 3.88 (2H, s, C-$7CH_2$), 4.15-4.40 (4H, m, C-$3CH_2$OMs), 4.80 and 5.12 (2H, ABq, J 15 Hz, C-$3CH_2$), 4.99 (1H, d, J 6 Hz, C-6H), 5.85 (1H, dd, J 6 and 8 Hz, C-7H), 6.73-7.02 (4H, m, C-4CH, C-7NH and 2 thiophene H) and 7.20-7.46 (11H, m, 1 thiophene and 10 phenyl H)

Intermediate 6

Diphenylmethyl 3-(bis-2-chloroethyl carbamoyl)-7$\beta$-(2-acetamido)-3-cephem-4-carboxylate

To a stirred solution of N-chloro succinimide (0.267g) in dry tetrahydrofuran (20ml) under a nitrogen atmosphere at room temperature was added triphenyl phosphine (0.525g) as a solution in tetrahydrofuran (5ml). Upon formation of a white precipitate Intermediate 4 (0.65g) was added as a solution in tetrahydrofuran (5ml). The reaction mixture was stirred at room temperature until the precipitate had disappeared. The mixture was poured into $NaHCO_3$ solution and extracted twice with dichloromethane. The combined organic fractions were dried over anhydrous magnesium sulphate, filtered and the solvent removed by evaporation in vacuo. Purification was by flash column chromatography, eluting with 5% ethyl acetate/dichloromethane to yield the title compound, (0.6g) $1^H$ nmr$\delta_H$ ($CDCl_3$): 3.26 and 3.47 (2H, ABq, J 18.5 Hz, C-$2H_2$) 3.42-3.70 (8H, m, ethyl H), 3.81 (2H, s, C-$7CH_2$), 4.82 and 5.13 (2H, ABq, J 12.5 Hz, C-$3CH_2$) 4.93 (1H, d, J 5.3 Hz, C-6H), 5.83 (1H, dd, J 5.3 and 8.6 Hz, C-7H), 6.86-7.08 (4H, m, C-4CH, C-7NH

and 2 thiophene H) and 7.20-7.42 (11H, m, 1 thiophene and 10 phenyl H)

Intermediate 7

Diphenylmethyl 3-(bis-2-bromoethyl carbamoyl)-7β-(2-acetamido)-3-cephem -4-carboxylate

To a stirred solution of Intermediate 4 (0.35g) in dry pyridine (10ml) at room temperature under a nitrogen atmosphere was added dibromotriphenylphosphorane (0.5g) as a solid in a single portion. The reaction mixture was stirred for 2 hours at room temperature and then poured into saturated $NaHCO_3$ solution. The product was extracted twice with dichloromethane and the combined organic fractions dried over anhydrous magnesium sulphate, filtered and the solvent removed by evaporation in vacuo. Purification was by flash column chromatography, eluting with 10% ethyl acetate/dichloromethane to yield the title compound (0.1g), $^1H$ nmr$\partial_H$ ($CDCl_3$): 3.25-3.75 (10H, m, C-2$H_2$ and ethyl H), 3.82 (2H, s, C-7$CH_2$) 4.82 and 5.14 (2H, ABq, J 13 Hz, C-3$CH_2$), 4.94 (1H, d, J 6 Hz, C-6H) 5.85 (1H, dd, J 6 and 9 Hz, C-7H) 6.76 (1H, d, J 9 Hz, C-7NH), 6.93 (1H, s, C-4CH), 6.96-7.00 (2H, m, 2 thiophene H), and 7.17-7.45 (11H, m, 1 thiophene and 10 phenyl H)

Intermediate 8

Diphenylmethyl 3-(bis-2-hydroxypropyl carbamoyl)-7β-(2-acetamido) -3-cephem-4-carboxylate

The title compound was prepared from Intermediate 3 and diisopropanol amine in a similar manner to Intermediate 4. $^1H$ nmr$\partial_H$ ($CDCl_3$); 1.05-1.19 (6H, m, methyl H), 3.12-3.69 (8H, m, C-2$H_2$ and N$CH_2$CH), 3.79 (2H, s, C-7$CH_2$), 4.72-5.12 (3H, m, C-6H and C-3$CH_2$), 5.82 (1H, dd, J 6 and 10 Hz, C-7H), 6.86-7.94 (4H, m, C-4CH, C-7NH and 2 thiophene H) and 7.13-7.45 (11H, m, 1 thiophene and 10 phenyl H)

Intermediate 9

Diphenylmethyl 3-(bis-2-chloropropyl carbamoyl)-7β-(2-acetamido) -3-cephem-4-carboxylate

The title compound was prepared from Intermediate 8 in a similar manner to Intermediate 6. $^1H$ nmr$\partial_H$ - ($CDCl_3$); 1.43 (3H, d, J 7 Hz, methyl), 1.51 (3H, d, J 7 Hz, methyl), 3.22-3.91 (8H, m, C-2$H_2$ and N$CH_2$CH), 3.83 (2H, s, C-7$CH_2$), 4.55-5.14 (2H, m, C-3$CH_2$) 4.98 (1H, d, J 6 Hz, C-6H) 5.84 (1H, dd J 6 and 8 Hz, C-7H), 6.85 (1H, d, J 8 Hz, C-7NH), 6.98-7.02 (3H, m, C-7NH and 2 thiophene H) and 7.32-7.47 (11H, m, thiophene and phenyl H).

Intermediate 10

Diphenylmethyl 3-(bis-2-bromopropyl carbamoyl)-7β-(2-acetamido) -3-cephem-4-carboxylate

The title compound was prepared in a similar manner to Intermediate 6 from Intermediate 8 and N-bromosuccinimide, $^1H$ nmr$\partial_H$ ($CDCl_3$); 1.48 (3H, d, J 7.5 Hz methyl H), 1.72 (3H, d, J 7.5 Hz methyl H), 3.23-3.95 (6H, m, C-2$H_2$ and N$CH_2$), 3.86 (2H, s, C-7$CH_2$), 4.34-4.48 (2H, m, CHBr), 4.70-5.22 (2H, m, C-3$CH_2$), 5.01 (1H, d, J 5 Hz, C-6H), 5.89 (1H, dd, J 5 and 8 Hz, C-7H), 6.32 (1H, d, J 8 Hz, C-7NH), 6.88-7.05 (3H, m, C-4CH and 2 thiophene H) and 7.26-7.48 (11H, m, thiophene and phenyl H).

Intermediate 11

Diphenylmethyl 3-(N,N bis(2-hydroxyethyl)-1,4-phenylenediamine carbamoyl-7β-(2-acetamido)-3-cephem-4-carboxylate

The title compound was prepared from Intermediate 3 and N,N-bis(2-hydroxyethyl)-1,4-phenylenediamine, in a similar manner to Intermediate 4. $^1H$ nmr$\partial_H$ ($CDCl_3$); 3.31-3.56 (6H, m, C-2$H_2$ and ArN$CH_2$), 3.73-3.85 (6H, m, C-7$CH_2$ and $CH_2$OH), 4.81 and 5.05 (2H, ABq, J 13 Hz, 3-$CH_2$), 4.91 (1H, d, J 5 Hz, C-6H), 5.82 (1H, dd, J 5 and 11 Hz, C-7H), and 6.57-7.46 (16H, m, C-7NH, NHAr, C-4CH, thiophene and phenyl H)

Intermediate 12

Diphenylmethyl 3-(N,N bis (2-chloroethyl)-1,4-phenylenediamine carbamoyl -7$\beta$-(2-acetamido)-3-cephem-4-carboxylate

The title compound was prepared from Intermediate 12 and dichlorotriphenylphosphorane, in a similar manner to Intermediate 7. $^1$H nmr$\delta$ $_H$($CDCl_3$); 3.34-3.75 (10H, m, C-2H $_2$and ethyl H), 3.87 (2H, s, C-7$CH_2$), 4.83-5.08 (2H ABq, J 13 Hz, C-3$CH_2$), 4.96 (1H, d, J 5 Hz, C-6H), 5.88 (1H, dd, J 5 and 10 Hz, C-7H), 6.65 (1H, d, J 10 Hz, C-7NH), 6.92-7.04 (3H, m, C-4CH and 2 thiophene H) and (11H, m, thiophene and phenyl H).

Intermediate 13

Diphenylmethyl 3-chloromethyl-7$\beta$-(2-thienylacetamido)-3-cephem-4 -carboxylate

To a stirred solution of Intermediate 2 (5.35g) in dry terahydrofuran under a nitrogen atmosphere at 0°C was added pyridine (1.25ml) and dimethylformamide (10µl, catalysis). Thionyl chloride (1.126ml) was added dropwise and the mixture stirred for 10 minutes at 0°C. It was then poured into saturated $NaHCO_3$ solution and the product extracted twice with dichloromethane. The combined organic layers were dried over anhydrous magnesium sulphate, filtered and the solvent removed by evaporation in vacuo. Purification was by flash column chromatography, eluting with 10% ethyl acetate/dichloromethane to yield the title compound (1.59g) $^1$H nmr$\delta_H$ ($CDCl_3$); 3.43 and 3.59 (2H, ABq, J 18 Hz, C-2$H_2$), 3.86 (2H, s, C-7$CH_2$), 4.40 (2H, s, C-3$CH_2$), 4.98 (1H, d, J 5 Hz, C-6H), 5.85 (1H, dd, J 5 and 9 Hz, C-7H), 6.35 (1H, d, J 9 Hz, C-7NH), 6.93 (1H, s, C-4CH), 6.95-7.05 (2H, m, thiophene H), and 7.23-7.46 (11H, M, thiophene and phenyl H).

Intermediate 14

3-Chloromethyl-7$\beta$-(2-thienylacetamido)-3-cephem-4-carboxylic acid

The title compound was prepared from Intermediate 13 in a similar manner to the compound of Example 1. $^1$H nmr$\delta_H$ (DMSO-$d_6$); 3.52 and 3.69 (2H, ABq, J 18 Hz, C-2$CH_2$), 3.82 (2H, s, C-7$CH_2$), 4.42 and 4.59 (2H, ABq, J 13 Hz, C-3$CH_2$), 5.07 (1H, d, J 5 Hz, C-6H), 5.82 (1H, dd, J 5 and (9 Hz, C-7H), 7.00-7.08 (2H, m, thiophene H), 7.32-7.40 (1H, m, thiophene H) and 9.12 (1H, d, J 9 Hz, C-7NH)

Intermediate 15

Diphenylmethyl 3-fluoroacetoxymethyl-7$\beta$-(2-thienylacetamido)-3-cephem -4-carboxylate

To a stirred solution of Intermediate 2 (1.42g) and pyridinium fluoroacetate (0.43g) in dry dichloromethane (5ml) at room temperature under a nitrogen atmosphere was added dicyclohexylcarbodiimide (0.564g). The reaction mixture was allowed to stir for 1 hour and then filtered, and the solvent removed by eveporation in vacuo to yield the title compound (1.4g) which was used without further purification. $^1$H nmr$\delta_H$ ($CDCl_3$): 3.37 (2H, d, J 4 Hz, C-3$CH_2$), 3.80 (2H, s, C-7$CH_2$), 4.70 (2H, d, J 48 Hz, $CH_2F$), 4.97 (1H, d, 5 Hz, C-6H), 5.85 (1H, dd, J 5 and 12 Hz, C-7H), 6.91 (1H, s, C-4CH), 6.70-7.00 (3H, m, C-7NH and 2 thiophene H) and 7.10-7.60 (11H, m thiophene and phenyl H)

Intermediate 16

Diphenylmethyl 3-(2-chloroethyl carbamoyl)-7$\beta$-(2 acetamido)-3-cephem-4-carboxylate

Intermediate 2 (0.97g) was dissolved in dry pyridine (10ml) at -23°C. To this was added 2-chloroethyl isocyanate (0.485ml) and the mixture stirred under nitrogen for 30 minutes after which the temperature was raised to room temperature and stirred for a further 4 hours. The pyridine was removed by evaporation in vacuo and the residue taken up in chloroform and washed with 1M HCl, saturated $NaHCO_3$ solution and water. The organic layer was dried with anhydrous magnesium sulphate, filtered and concerntrated in vacuo. The product was purified by flash column chromatography eluting with 15% ethyl acetate/$CH_2Cl_2$. The pure $\Delta$3 isomer was isolated by crystallisation from ethyl acetate/petroleum ether to give the title compound (0.633g). $^1$H nmr$\delta_H$ ($CDCl_3$): 3.32-3.59 (6H, m, C-2$CH_2$ and ethyl H), 3.86 (2H, s, C-7$CH_2$), 4.98

(1H, d, J 4.2 Hz, C-6H), 4.78 and 5.05 (2H, ABq, J 13.4 Hz, C-3$CH_2$), 5.88 (1H, dd, J 4.2 and 9.2 Hz, C-7H), 6.33 (1H, d, J 9.2 Hz, C-7NH), 6.95-7.03 (3H, m, C-4CH and thiophene H) and 7.27-7.45 (11H, m, thiophene and phenyl H).

Example 1

3-(Bis-2-methanesulphonylethyl carbamoyl)-7β-(2-acetamido)-3-cephem-4-carboxylic acid

To a stirred solution of Intermediate 5 (0.076g) in $CH_2Cl_2$ (10ml) at room temperature was added 0.5ml of anisole followed by 0.5ml of trifluoroacetic acid. The mixture was stirred for 2 hrs and then $iPr_2O$ (20ml) added and the solvent removed by evaporation in vacuo. Purification was by reverse phase HPLC eluting with 0.1% trifluoroacetic acid and 0.1% MeCN to yield the title compound (0.049g) $^1H$ nmr$\delta_H$ ($CDCl_3$): 3.08 (6H, s, $OSO_2CH_3$), 3.42-3.70 (6H, m, C-$2H_2$ and C-3N$CH_2$), 3.79 (2H, s, C-7$CH_2$), 4.22-4.37 (4H, m, C-3$CH_2$OMs), 4.80 and 5.07 (2H, ABq, J 12.9 Hz, C-3$CH_2$), 5.01 (1H, d, J 5.4 Hz, C-6H), 5.79 (1H, dd, J 5,4 and 8.6 Hz, C-7H), 6.92-7.00 (2H, m, 2 thiophene H) and 7.25-7.40 (2H, m, C-7NH and 1 thiophene H).

Example 2

3-(Bis-2-chloroethyl carbamoyl)-7β-(2-acetamido)-3-cephem-4-carboxylic acid

The title compound was prepared in a similar manner to the compound of Example 1 from Intermediate 6. $^1H$ nmr$\delta_H$ ($D_3COD$): 3.43-3.72 (10H, m, C-$2H_2$ and ethyl H), 3.82 (2H, s, C-7$CH_2$), 4.86 and 5.20 (2H, ABq, J 15 Hz, C-3$CH_2$), 5.08 (1H, d, J 6 Hz, C-6H), 5.73 (1H, d, J 6 Hz, C-7H), 6.92-7.00 (2H, m, thiophene H), 7.27 (1H, d, J 7 Hz, thiophene H) and 9.19 (1H, d, J 7.5 Hz, C-7NH).

Example 3

3-(Bis-2-bromoethyl carbamoyl)-7β-(2-acetamido)-3-cephem-4-carboxylic acid

The title compound was prepared in a similar manner to the compound of Example 1 from Intermediate 7. $^1H$ nmr$\delta_H$ (DMSO-$d_6$); 3.25-3.75 (10H, m, C-$2H_2$ and ethyl H), 3.79 (2H, s, C-7$CH_2$), 4.71 and 5.06 (2H, ABq, J 13 Hz, C-3$CH_2$) 5.10 (1H, d, J 6 Hz, C-6H), 5.70 (1H, dd, J 6 and 9 Hz, C-7H), 6.90-7.00 (2H, m, 2 thiophene H), 7.37 (1H, d, J 5 Hz, thiophene H) and 9.15 (1H, d, J 9 Hz, C-7NH).

Example 4

3-(Bis-2-chloropropyl carbamoyl)-7β-(2-acetamido)-3-cephem-4-carboxylic acid

The title compound was prepared from Intermediate 9 in a similar manner to the preparation of the compound of Example 1. $^1H$ nmr$\delta_H$ ($D_3COD$); 1.49 (6H, d, J 7.5 Hz, methyl), 3.47-3.78 (8H, m, C-$2H_2$ and N$CH_2$CH), 3.80 (2H, s, C-7$CH_2$), 4.82-5.21 (2H, m, C-3$CH_2$), 5.10 (1H, d, J 6 Hz, C-6H), 5.53 (1H, d, J 6 Hz, C-7H), 6.93-6.98 (2H, m, thiophene H), 7.28 (1H, d, J 7.5 Hz, thiophene) and 9.22 (1H, d, J 8Hz, C-7NH).

Example 5

3-(Bis-2-bromopropylcarbamoyl)-7β-(2-acetamido)-3-cephem-4-carboxylic acid

The title compound was prepared in a similar manner to the compound of Example 1 from Intermediate 10. $^1H$ nmr$\delta_H$ ($CD_3CN$); 1.5 (6H, d, J 5 Hz, methyl H), 3.43-3.72 (6H, m, C-$2H_2$ and N$CH_2$), 3.82 (2H, s, C-7$CH_2$), 4.42-4.49 (2H, m, CHBr), 4.78-5.12 (2H, ABq, J 14 Hz, C-3$CH_2$), 5.04 (1H, d, J 4 Hz, C-6H), 5.78 (1H, dd, J 4 and 9 Hz, C-7H), 6.85-7.27 (3H, m, thiophene H) and 8.04 (1H, d, J 9 Hz, C-7NH).

Example 6

3-(N,N bis(2-chloroethyl)-1,4-phenylenediamine carbamoyl)-7β-(2-acetamido)-3-cephem-4-carboxylic acid

The title compound was prepared in a similar manner to the compound of Example 1 from Intermediate 12. $^1H$ nmr$\delta_H$ ($D_3COD$); 3.52-3.73 (10H, m, C-$2H_2$ and ethyl H), 3.79 (2H, s, C-7$CH_2$), 4.72 and 5.06 (2H,

ABq, J 14 Hz, C-3$CH_2$), 5.16 (1H, d, J 5 Hz, C-6H), 5.72 (1H, d, J 5 Hz, C-7H), 6.68 (2H, d, J 8 Hz, phenyl H), 6.93-6.99 (2H, m, thiophene H) and 7.25-7.34 (3H, m, thiophene and phenyl H).

Example 7

3-(Purin-6-ylthio methyl)-7$\beta$-(2-acetamido)-3-cephem-4 carboxylic acid

To a stirred solution of Intermediate 4 (0.186g) and 6-mercaptopurine (0.085g) in dry dimethylformamide (2ml) at room temperature was added 1,8-diazabicyclo[5.4.0)]undec-7-ene (0.15ml). After 15 minutes the crude reaction mixture was purified by reverse phase HPLC eluting with 0.1% trifluoroacetic acid (TFA) $H_2O$/0.1% TFA/MeCN, to yield the title compound (0.8g) $^1H$ nmr$\partial_H$ (DMSO-$d_6$): 3.57 and 3.79 (2H, ABq, J 18 Hz, C-2$H_2$), 3.77 (2H, s, C-7$CH_2$), 4.11 and 4.90 (2H, ABq, J 13 Hz, C-3$CH_2$), 5.08 (1H, d, J 5 Hz, C-6H), 5.68 (1H, dd, J 5 and 9 Hz, C-7H), 6.90-6.97 (2H, m, thiophene H) 7.32 (1H, d, J 3 Hz, thiophene H), 8.42 (1H, s, purine H), 8.67 (1H, s, purine H) and 9.10 (1h, d, J 9 Hz, C-7NH).

Example 8

3-((5-Fluorouracil)methyl)-7$\beta$-(2-acetamido)-3-cephem-4-carboxylic acid

The title compound was prepared in a similar manner to the compound of Example 7 from Intermediate 14 and 5-fluorouracil. $^1H$ nmr$\partial_H$ (DMSO-$d_6$): 3.41 and 3.53 (2H, ABq, J 18 Hz, C-2$H_2$), 3.78 (2H, s, C-7$CH_2$), 4.26 and 4.93 (2H, ABq, J 15 Hz, C-3$CH_2$), 5.07 (1H, d, J 4 Hz, C-6H), 5.61 (1H, dd, J 4 and 9 Hz, C-7H), 6.89-6.95 (2H, m, thiophene H), 7.34 (1H, d, J 3 Hz, thiophene H), 7.94 (1H, d, J 7.5 Hz, uracil H) and 9.13 (1H, d, J 9 Hz, C-7NH).

Example 9

3-(Guanin-6-ylthiomethyl)-7$\beta$-(2-acetamido)-3-cephem-4-carboxylic acid

The title compound was prepared in a similar manner to the compound of Example 7 from Intermediate 14 and 6-thioguanine. $^1H$ nmr$\partial_H$ (DMSO-$d_6$): 3.52 and 3.63 (2H, ABq, J 18 Hz, C-2$H_2$), 3.64 (2H, s, C-7$CH_2$), 4.02 and 4.71 (2H, ABq, J 15 Hz, C-3$CH_2$), 5.10 (1H, d, J 5 Hz, C-6H), 5.65 (1H, dd, J 5 and 8 Hz, C-7H), 6.88-6.97 (2H, m, thiophene H), 7.30-7.38 (1H m, thiophene H), 7.95 (1H, s, guanine H) and 9.12 (1H, d, J 8 Hz, C-7NH).

Example 10

3-Fluoroacetoxymethyl-7$\beta$-(2-thienylacetamido)-3-cephem-4-carboxylic acid

The title compound was prepared from Intermediate 15 in a similar manner to the compound of Example 1. $^1H$ nmr$\partial_H$ (DMSO-$d_6$): 3.51 and 3.62 (2H, ABq, J 18 Hz, C-2$H_2$), 3.77 (2H, s, C-7$CH_2$), 4.82 and 5.18 (2H, ABq, J 15 Hz, C-3$CH_2$), 5.04 (2H, d, J 37.5 Hz, $CH_2F$), 5.10 (1H, d, J 5 Hz, C-6H), 5.72 (1H, dd, J 5 and 8 Hz, C-7H), 6.88-6.97 (2H, m, thiophene H), 7.47 (1H, d, J 4 Hz, thiophene H) and 9.14 (1H, d, J 8 Hz, C-7NH).

Example 11

3-(2-chloroethylcarbamoyl)-7$\beta$-(2-acetamido)-3-cephem-4-carboxylic acid

The title compound was prepared in a similar manner to the compound of Example 1 from Intermediate 16. $^1H$ nmr$\partial_H$ ($D_3CN$): 3.36-3.66 (6H, m, C-2$H_2$ and ethyl H), 3.78 (2H, s, C-7$CH_2$), 4.77 and 4.93 (2H ABq, J 14.4 Hz, C-3$CH_2$), 5.05 (1H, d, J 5.51 Hz, C-6H) 5.75 (1H, dd, J 5.1 and 8.9 Hz, C-7H), 6.00 (1H, s, C-3NH), 6.92-7.01 (2H, m, thiophene H) and 7.24-7.36 (2H, m, C-7NH and thiophene H).

Example 12

3-(2-chloroethyl N-nitroso carbamoyl)-7β-(2-acetamido)-3-cephem-4-carboxylic acid

A slurry of anhydrous sodium acetate (0.579g) in dry dichloromethane (5ml) was cooled to -78°C. $NO_2$ was bubbled through dichloromethane (10ml) to give a solution of $N_2O_4$ (1.31g). This was added to the sodium acetate slurry.

The compound of Example 11 (0.315G) was suspended in dichloromethane (15ml) and was dissolved by addition trifluoroacetic acid (0.3ml). The resulting solution was cooled to 0°C and added dropwise to the solution $N_2O_4$. After 15 minutes the reaction mixture was warmed to -23°C and stirred for a further 20 minutes. The solvent was removed by evaporation in vacuo and the residue triturated with isopropyl ether. The residue was taken up in chloroform, washed with brine, dried with anhydrous magnesium sulphate and concerntrated in vacuo. The product was purified by reverse phase HPLC. eluting with 0.1% trifluoroacetic acid TFA.acetonitrile, to yield the title compound. $^1H$ nmr$\delta_H$ ($CD_3CN$): 3.56 (2H, t, ethyl H), 3.54 and 3.71 (2Hm ABQ, J 17.3 Hz, C-$2H_2$), 3.78 (2H, s, C-$7CH_2$), 4.06 (2H, t, ethyl H), 5.07 (1H, d, J 5.1 Hz, C-6H), 5.17 and 5.47 (2H ABq, J 13.3 Hz, C-$3CH_2$), 5.79 (1H, dd, J 5.1 and 9.2 Hz, C-7H) 6.94-7.01 (2H, m, thiophene H) and 7.27-7.34 (2H, m, C-7NH and thiophene H).

Example 13

β-Lactamase Catalysed Cleavage of Prodrug to Drug

The ability of various β-lactamases to catalyse the hydrolysis of the prodrugs prepared in the above Examples was tested.

Purified β lactamases from several microorganisms were tested namely: Enterobactor cloacae p99, E.coli RTEM; Bacillus cereus I and II; and Staphylococcus aureus.

The method used was as follows:

Each prodrug (0.05ml of a 10mM solution in 50mM phosphate buffer, pH7.0) was mixed with phosphate buffer (0.95ml; 50mM; pH7.0) and the solution scanned using a UV-VIS spectrophotometer in the wavelength range 200-500nm. β-Lactamase (0.01ml of a 1 $mg.ml^{-1}$ solution in 50mM phosphate buffer, pH7.0) was mixed with the solution, which was then incubated at 37°C, and scanned, as previously, at 5 minute intervals.

In addition, 6-mercaptopurine and 6-thioguanine prodrug (Example 7 and 9) hydrolysis could be followed by an increase in absorbance at 323 and 340nm respectively. In the case of the 5-fluorouracil prodrug (Example 8) the spectrum of the drug portion, 5-fluorouracil, interfered with the change in absorbance of the β-lactam ring. Cleavage of the β-lactam ring was therefore followed by use of a stopped assay which detects the cephalosporic acid by reaction with copper sulphate and neocuproine as follows:

10μl 50μM-1mM prodrug in DMSO
10μl β-Lactamase
80μl 50mM phosphate buffer pH7.0

were incubated at 37°C for 30 min

400μl 50mM phosphate buffer pH7.0
500μl solution C (equal volumes of solutions A and B, see below)

were then added. After 2 hrs, the absorbance of the solutions at 450nm was determined.

[Solution A: 16mg neocuproine - HCl in 1.2 ml $H_2O$ + 18.8 ml 0.2M Na acetate pH4.75 + 10g/l sodium dodecyl sulphate (SDS)
Solution B: 40mg $CuSo_4$. $5H_2O$ in 20ml 0.2M sodium acetate pH4.75 + 10 g/l SDS
Solution C: equal volumes of A and B]

Evidence for release of the drug from the prodrug was obtained by following the enzyme/prodrug reaction by NMR and comparing the spectrum of the product formed with the known spectrum of the prodrug. In the case of the 6-mercaptopurine and 6-thioguanine prodrugs this was unnecessary as the UV-VIS spectra of the drugs are distinctive and identical to the reaction products.

Rates of hydrolysis were determined in the case of the mustard prodrugs (Examples 2,3,4 and 5)by following the decrease in absorbance at 265nm with time after addition of 0.1-10 μg/ml β-Lactamase to 0.1mg/ml prodrug in buffer

6-mercaptopurine was followed at 323nm
6-thioguanine was followed at 340 nm

The rate of hydrolysis of selected prodrug by β-lactamase is give below in Table 1.

Table 1

| Enzyme Source | Prodrug - Compound of Example No. | kcat/Km ($M^{-1}5^{-1}$ | kcat ($5^{-1}$) | Km (μM) |
|---|---|---|---|---|
| E.cloacae | 2<br>3<br>5 | $4.7 \times 10^6$<br>$2.1 \times 10^6$<br>$3.0 \times 10^6$ | 515<br>151<br>120 | 109<br>73<br>40 |
| E.coli | 4<br>7 | $1.5 \times 10^5$<br>$5.9 \times 10^4$ | 10.5<br>8.1 | 70<br>138 |
| B.cereus II | 4<br>3 | $6.3 \times 10^4$<br>$5.5 \times 10^4$ | 3.4<br>3.5 | 54<br>63 |

In each instance cleavage of the β-Lactam ring was observed, with release of the drug from the prodrug.

Example 14

Antibody-β-lactamase hybrid Proteins produced by Recombinant DNA technology

Antibody enzyme hybrids may be produced by recombinant DNA means whereby the DNA encoding at least the antigen recognition elements of an antibody is fused to that of the DNA encoding at least the active portion of an enzyme. This DNA sequence together with that of the complementary light or heavy chain of the antibody is than expressed in E.coli, yeast or mammalian cells using appropriate expression vectors and the antibody-enzyme hybrid produced purified and characterised by the standard techniques of protein biochemistry and immunology.

In order to produce a hybrid suitable for the demonstration of the principle of antibody directed cleavage of a prodrug, DNA encoding for the heavy chain CHI and hinge region of the antibody anti NP (nitro iodophenyl antigen) was fused to DNA encoding the entire β-lactamase gene. This was expressed (together with the corresponding light chain gene) using an appropriate vector in a transient expressing mammalian COS cell system. The resulting protein produced was shown to consist of the assembled light and heavy chain-β-lactamase fusion. The anti NP-β-lactamase enzyme hybrid was further shown to be capable of both recognising the specific hapten nitroiodophenol and exhibiting β-lactamase activity.

The anti-NP β-lactamase hybrid protein was produced as folllows

(1) Construction of COS cell Expression Vector

Plasmid EE7.H3 [International Patent Application WO89/12098] was restricted with HindIII and SmaI and then treated with alkaline phosphatase.

PSV.VnG 2b Δ(CH2, CH3), a known plasmid having an operon encoding the heavy chain variable domain, first constant domain and hinge region of an anti-NP (nitroiodophenyl) antigen, was digested with XhoI and incubated with T4 DNA polymerase to fill In the XhoI site. After removal of the T4 DNA polymerase activity, the DNA was restricted with HindIII and the largest fragment [HindIII-Xho(blunt)] of coding sequence for the first constant domain and hinge region of the antibody was inserted between the HindIII and SmaI site of the restricted and treated EE7.H3 plasmid. This produced the plasmid EE7.CH1H and recreated a unique XhoI site.

PSV.VnG 2b Δ(CH2, CH3) was also digested with NcoI and the synthetic oligonucleotide 5'CATGGTGGAAGCTTCCAC3' was ligated thereto. This reformed the NcoI site with Kozak consensus sequence (CCACC) around the initiator ATG and placed a HindIII site upstream. Digestion of the ligation product with HindIII allowed the isolation of a HindIII fragment containing the variable domain of NP gene (Approx. 1100bp). This HindIII fragment was cloned into the HindIII site of EE7.CH1H to produce the plasmid EE7.VCH1H.

Into this vector was cloned the K2SP domain from TPA as described in International Patent Specification No. WO89/12098. This generated the plasmid termed EE7.VCH1H.K2SP. The HCMV promoter was isolated on a 2.1kb HindIII fragment from EE6.HCMV (International Patent Specification No. 89/01036) and

cloned into EE7.VCH1H.K2SP that had been partially digested with HindIII. A plasmid was isolated that contained the HCMV fragment in the correct orientation and upstream of the V region of the anti-NP gene. This plasmid is EE7.VCH1H.K2SP.HCMV. This plasmid was used to construct the β-lactamase anti-NP fusion by exchanging the K2SP fragment, which could be isolated on a XhoI fragment.

Construction of a suitable β-lactamase gene to clone into EE7.VCH1H.K2SP.HCMV

A commercially available plasmid [hereinafter termed pPOD2353] containing most of the E.coli RTEM β-lactamase gene was obtained. The 5' sequence was as follows:

```
                                      R1
5'AAGCTTGATATCGAATTCCAGCTTGCCCCCCAGAAACG
                      P  A  C  P  P  E  T
```

The sequence AATT is the recognition sequence for the restriction enzyme Eco.R1
The region underlined is the N terminal amino acid sequence of the mature processed β-lactamase starting at residue 2. The correct N terminal sequence for β-lactamase is given below:
H P E T L.....
In order to clone this gene three different modifications had to be performed.

a) An XhoI site was added to the 5' end of the gene
b) The DNA sequence coding for the N terminal His was added to the gene pPOD2353
c) An XhoI site was added to the 3' end of the gene

The above manipulations were performed using appropriate synthetic oligonucleotides as primers for the polymerase chain reaction (PCR). The sequence for the forward oligonucleotide in the PCR is
5'GGGGGCTCGAGCACCCAGAAACGCTGGTGAAA 3'
The above oligo nucleotide places a XhoI site immediately upstream of the first codon of the fully processed β-lactamase gene, such that the sequence through the junction will be -CH2/β-lac:
..........NLEHPETL..........
The sequence NLE is the C terminal sequence of the CH2 domain of the anti-NP antibody, and the remaining sequence is the N terminal sequence of β-lactamase.

The sequence for the "reverse" oligonucleotide in the PCR is:
5'GGGGGCTCGAGTTTTAAATCAATCTAAAGTAT 3'
The above oligonucleotide places a XhoI site downstream from the natural stop codon from β-lactamase from plasmid pPOD2353.

The product from the PCR was restricted with XhoI and this fragment was purified using standard protocols.

The plasmid EE7.VCH1H.K2SP.HCMV was restricted with XhoI which resulted in the loss of the K2SP gene and the plasmid treated with alkaline phosphatase. Into this vector was cloned the β-lactamase XhoI cut PCR product (described above). A plasmid (pNPSTCH) was isolated that contained the β-lactamase fragment in the correct orientation.

(2) Expression of pNPSTCH

The plasmid pNPSTCH was expressed in a transient expression system involving transfection into COS-1 cells. COS-1 cells are derived from CV1 monkey kidney cells (Gluzman, 1981) which have been transfected with the SV40 virus with its origin of replication deleted. The COS-cell transfection procedure provides a rapid, and easily reproducible method for producing, analytical quantities of protein for biochemical characterisation [Whittle et al (1987), Protein Engineering, 1, 499-505] Biosynthetic radiolabelling of transfected cells, followed by purification and visualisation by SDS PAGE can deomonstrate that the gene product is correctly transcribed and translated to generate a polypeptide of the expected size.

The plasmid pNPSTCH was co-transfected, with a plasmid containing the anti-NP light chain gene (pNPLC), into COS-1 cells. Following incubation at 37°C for 72h the cell supernatents were assayed for β-lactamase activity. Determination of enzyme activity of the β-lactamase constructs was performed by addition of cell supernatant to 1mM cephalothin in 50mM phosphate buffer pH7, and following the decrease in absorbance at 265nm. Quantification was performed by determining the rates of hydrolysis by known concentrations of E.coli RTEM β-lactamase in the same medium.

A blank assay (containing no cell supernatant) and a control supernatant from COS cells transformed with a plasmid encoding a NP fusion with a portion of the tPA enzyme (termed aNIPK2sp) showed essentially no β-lactamase activity. However, a supernatant from COS cells transformed with PNPSTCH and PNPLC showed β-lactamase activity (equivalent to approximately 0.1μg/ml enzyme).

The ability of the NP-β-lactamase fusion to bind NP antigen was analysed. Expressed COS cell proteins were labelled for 48h following transfection, with [$^{35}$S]methionine (Whittle et al, 1987 ibid) Supernatents from the COS cells were incubated overnight with Sepharose beads coupled to NP. After washing the beads, bound proteins were separated on an SDS-10% PAGE under reducing and non-reducing conditions. The gel was treated with an autoradiography enhancer (Amplify, Amersham International) dried and exposed to X-ray sensitive film. The molecular weights of the various fragments which might be generated are given below

| | |
|---|---|
| - β-lactamase | (approx 30K) |
| - Light chain | (approx 28K) |
| - Fd + hinge | (approx 30K) |
| - Fd + hinge + β-lactamase | (approx 60K) |

If correct expression is taking place then the predicted distribution of bands from samples analysed under reducing conditions are, free light chain (30K), and a band of 60K which corresponds to the heavy chain with β-lactamase attached. The samples treated under non-reducing conditions are expected to give two different species:

(1) $F(ab)_2$-like molecules with 2 molecules of β-lactamase attached

(2) Fab' molecules with β-lactamase attached.

From the result obtained it was clear that the transformed COS-1 cells were producing molecules which were capable of binding to NP, and that all of the species which were predicted above to be made from the genes were present.

A formal possibility remains that the β-lactamase activity observed in the COS supernatants is from free β-lactamase arising from enzymatic cleavage from the antibody and that the measured pro-drug activity is from free β-lactamase.

In order to demonstrate that the activity observed in the pro-drug assay was due to β-lactamase attached to antibody, an assay was performed in which 2 identical COS cell supernatents expressing NP-β-lactamase were treated as detailed below:

To one of the samples NP-sepharose was added and left incubating at room temperature. The second sample was incubated without NP sepharose at room temperature. Both samples were then centrifuged and identical volumes removed and assayed for pro-drug activation. It was observed that incubation of COS cell supernatants with NP sepharose depleted β-lactamase activity thus demonstrating the fusion of antigen binding and β-lactamase activities.

## Example 15

### Cell tissue culture assay of relation drug/prodrug toxicity

A tissue culture assay using a mouse lymphoma cell line [Phillips (1974), Biochem. Pharmacol. 23, 131-138] was used to determine the relative toxicity of drug and prodrug samples. The cepholosporin - prodrugs tested were those of Example 2,5,6 - 10 and 12.

For compounds of Examples 2, and 7-10 the corresponding non-cephalosporin linked drug was tested. In the case of compounds of Examples 5, 6 and 12 the drug portion of the prodrug was generated in situ in the assay by the addition of the enzyme β-lactamase as indicated below.

### Methodology

(i) Cell line

Mouse lymphoma cells, line L5178Y clone 3.7.2.C, was used for these studies. This clone was derived from the L5178Y wild-type line and is heterozygous for the thymidine kinase locus. It is widely used for assays of mutation to thymidine analogue resistance (loss of thymidine kinase). Stocks were maintained frozen in liquid nitrogen and recovered 7 days before the start of the study.

(ii) Culture conditions

The cells were grown in RPMI 1640 medium buffered with 25 mM HEPES, and supplemented with 10% horse serum, 1mM sodium pyruvate, and 0.05% Pluriol (PF 6800 from BASF). In most cases, penicillin (100 iu/ml) and streptomycin (100µg/ml) were also included in the medium. Stock cultures were maintained by subculture into fresh medium every 2-3 days. Incubation was at 37°C in an atmosphere of 5% $CO_2$ in air. Under these conditions, the cells grew as a static cell suspension with a population doubling time (cell cycle time) of 10-12 hr.

(iii) Cytotoxicity assay

A growing culture, at a cell density of 1-2 x $10^6$/ml, was diluted with fresh medium to a density of 5 x $10^5$ cells/ml. An appropriate number of universal bottles was prepared with 4.5ml medium and O.5ml cell suspension to give a final cell concentration of 5 x $10^4$ cells/ml. In some tests, the cells were centrifuged and resuspended in Hank's Balanced Salt Solution (HBSS) to allow treatment in a simpler medium.

Test solutions were added in dimethylsulphoxide DMSO (final concentration 0.5% for continuous exposure or 1% for 1 hr exposure) and controls received the same amount of DMSO. Where applicable, β-lactamase was added (50µl of a 1mg/ml solution in phosphate buffered saline).

For 1 hr exposure of the cells, the universal bottles were incubated on a rotary mixer (2 revs per min) at 37°C for 1 hr and the medium changed to remove the test compound. In other cases, the treated cell suspensions were plated out immediately. At least 4 wells (15mm diameter) of Nunclon[R] 24-well plastic multidishes were inocculated with 1ml each from each universal bottle. The plates were incubated at 37°C.

The cell number per well at the start of incubation was checked by pooling the remaining suspension from each universal and counting a sample with a Coulter[R] electronic particle counter. Counts were made on two wells per treatment after incubation of the plates for 24, 48 or 72 hr. The contents of each well were mixed thoroughly with a Pasteur pipette to break up cell clumps and diluted 1 in 10 before counting twice with the Coulter counter.

(iv) Calculation of $ID_{50}$

Cell growth was expressed as the number of population doublings (PD) achieved during a given time period ( t hrs ), calculated as follows:

$$PD = \frac{\text{Log (mean count at t hrs} \div \text{mean count at o hrs)}}{\text{Log 2}}$$

(v) Calculations

Growth relative to control ( % G ) was calculated for each treatment as follows:

$$\%\,G = \frac{\text{PD (Treated)}}{\text{PD (Control)}} \times 100$$

β-Lactamase

A 1mg/ml stock solution of β-lactamase (RTEM) was prepared in 50mM phosphate buffer pH7 containing 0.2% azide. For assays of drug toxicity β-lactamase at a final concentration of 10mg/ml was

utilised. At this concentration there was complete conversion of prodrug to drug within 5 minutes.

RESULTS

Differential toxicity between prodrug and drug (assayed as described above) were up to 13 fold with $ID_{50's}$ in the micro molar range. Typical results, from the cephalosporin-thioguanine prodrug [compound of Example 9] drug system, are given below in Table 2.

**Table 2**

| Compound | Mr | ID50)µ (M) | Differential Toxicity (ID50 Prodrug/drug) |
|---|---|---|---|
| (i) Cephalosporin - thioguanine (Prodrug) | 504 | 6.44 ) | 13 |
| (ii) Thioguanine (drug) | 167 | 0.53 ) | |

This demonstrates a clear differential toxicity between prodrug and drug in this cephalosporin - thioguanine (Prodrug) /thioguanine (drug system), which was also observed in the other prodrug/drug systems tested in this Example.

The results thus demonstrate the suppression of the characteristic toxicity of each drug when incorporated in the prodrug structure.

**Claims**

1. A product for delivering a drug at a host target site, the product comprising an immunoconjugate and a prodrug as a combined preparation for sequential use in therapy, said immunoconjugate being capable of recognising and binding to one or more epitopes associated with the host target site and having a β-lactamase action capable of hydrolysing said prodrug to active drug or an unstable precursor thereof at the target site, characterised in that said immunoconjugate comprises a β-lactamase enzyme or fragment thereof directly linked to an antibody or antibody fragment capable of recognising and binding to the said one or more epitopes, and the prodrug is a cephalosporin or penicillin derivative of formula (3)

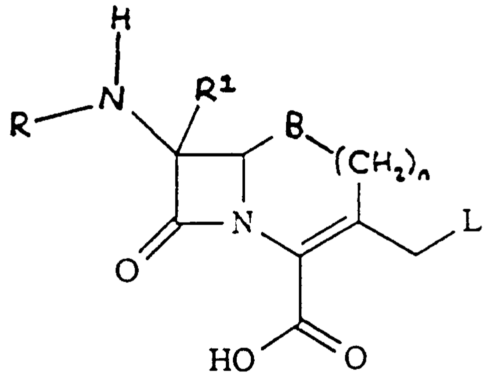

(3)

wherein R is an acyl or alkyl radical: $R^1$ is a hydrogen atom or an alkoxy group; B is $-CH_2-$, -O-, or -S-, n is zero or an integer 1 to 4 inclusive, L is the active drug or unstable precursor thereof and L is linked to the remainder of the molecule such that it forms a leaving group, whereby L is eliminated on hydrolysis of the prodrug.

2. A product according to claim 1 wherein L in formula (3) is linked to the remainder of the molecule through an oxygen, nitrogen or sulphur atom present in L.

3. A product according to claim 1 or 2 wherein L in formula (3) is a group $-O-CO-L^1$ or $-S-L^1$ where $L^1$ is the remainder of the drug or unstable precursor thereof.

4. A product according to claim 1, 2 or 3 wherein the host target site is a tumour, and L in formula (3) is an antineoplastic agent or an unstable precursor thereof.

5. A product according to claim 4 wherein the antineoplastic agent is an alkylating agent, an antimetabolite, an antibiotic, a mitotic inhibitor, an alkaloid, a hormone, a urea, a hydrazine or an imidazole.

6. A product according to claim 5, wherein the antineoplastic agent is an alkylating agent selected from chlorambucil, melphalan, mechlorethamine, cyclophosphamide, uracil mustard, triethylenephosphoramide, triethylenethiophosphoramide, busulphan or cisplatin.

7. A product according to claim 5, wherein the antineoplastic agent is an antimetabolite selected from methotrexate, fluorouracil and floxuridine, cytarabine, mercaptopurine, thioguanine, fluoroacetic acid or fluorocitric acid.

8. A product according to claim 5, wherein the antineoplastic agent is an antibiotic selected from bleomycin sulphate, doxorubicin, daunorubicin, mitomycin C, dactinomycin, plicamycin, calicheamicin, or esperamicin.

9. A product according to claim 5, wherein the antineoplastic agent is a mitotic inhibitor selected from etoposide, vincristine or vinblastine.

10. A product according to claim 5, wherein the antineoplastic agent is the alkaloid ellipticine.

11. A product according to claim 5, wherein the antineoplastic agent is a hormone selected from dromostanolone, testolactone, megestrol acetate, medroxyprogesterone acetate, diethylstilbestrol diphosphate, polyestradiol phosphate, estramustine phosphate or tamoxifen.

12. A product according to claim 5, wherein the antineoplastic agent is hydroxyurea.

13. A product according to claim 5, wherein the antineoplastic agent is the hydrazine procarbazine.

**14.** A product according to claim 5, wherein the antineoplastic agent is the imidazole dacarbazine.

**15.** A product according to any preceding claim wherein the $\beta$-lactamase enzyme or active fragment thereof is from Eschericia, Staphylococci, Pseudomonas, Bacteriodes, Klebsiella, Citrobacter, Bacillus, Enterobacter or Streptococci.

**16.** A product according to claim 15 wherein the $\beta$-lactamase enzyme or active fragment thereof is from B. cereus, Enterobacter cloacae or E. coli.

**17.** A compound of formula (3)

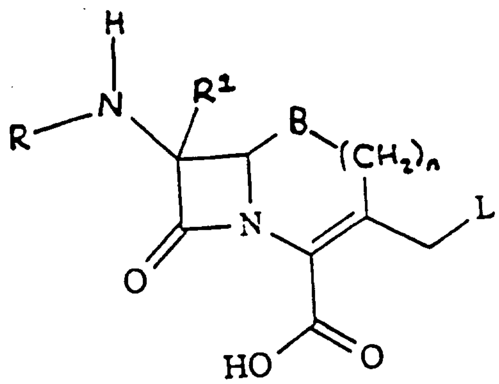

(3)

wherein R is an acyl or alkyl radical, $R^1$ is a hydrogen atom or an alkoxy group; B is $-CH_2-$, -O- or -S-; n is zero or an integer 1 to 4 inclusive and L is linked to the remainder of the molecule such that it forms a leaving group, characterised in that L is an antineoplastic agent being an alkylating agent; an antimetabolite; an antibiotic; a mitotic inhibitor; an alkaloid; a hormone; a urea; a hydrazine; or an imidazole, and provided that when $R^1$ is a hydrogen atom, B is -S- and n = 1, then L is not a daunomycin, an adriamycin, a methotrexate, a mitomycin, a bleomycin, melphalan, 6-mercaptopurine, cytosine arabinoside, a podophyllotoxin, a vinca drug, a difluoronucleoside, a sulfonylurea compound, a nitrogen mustard, a pteridine drug, a trichothecane, a colchicine or 5-fluorouracil.

**18.** A compound as claimed in claim 17 wherein B is -S- and n is an integer 1.

**19.** A compound as claimed in claim 17 or 18 wherein $R^1$ is a hydrogen atom.

**20.** A compound according to any of claims 17-19 wherein the antineoplastic agent L is linked to the rest of the molecule through an oxygen, nitrogen or sulphur atom present in L.

**21.** A compound as claimed in claim 20 wherein the antineoplastic agent L is $-O-CO-L^1$ where $L^1$ is the remainder of the antineoplastic agent or unstable precursor thereof.

**22.** A compound as claimed in claim 21 wherein the antineoplastic agent L is a group $-O-CO-L^1$ and $L^1$ is a group $-NR^6R^7$ wherein $R^6$ and $R^7$ are the same or different and is each a hydrogen atom or an optionally substituted $C_{1-6}$ alkyl group with the proviso that only one of $R^6$ and $R^7$ is a hydrogen atom; or $L^1$ is a group $-P-NR^6R^7$ where P is a phenyl group.

**23.** A compound as claimed in claim 21 wherein L is a thioguanine group.

**24.** A compound as claimed in any of claims 17-23 wherein R is an optionally substituted aliphatic, heteroaliphatic, aromatic, heteroaromatic, araliphatic or heteroaraliphatic carboxylic or carbothioic acid radical or a carbamoyl radical.

**25.** A compound as claimed in claim 24 wherein R is a group $R^2C=X$ where X is an oxygen or sulphur atom and $R^2$ represents a hydrogen atom or an optionally substituted group selected from amino, substituted amino, $C_{1-6}$ alkyl, $C_{1-6}$ alkylthio, $C_{6-12}$ arylthio, $C_{1-6}$alkoxy, $C_{6-12}$aryloxy, $C_{2-6}$ alkenyl or

alkynyl, aryl, ar$C_{1-3}$ alkyl, $C_{3-6}$ cycloakyl, $C_{4-10}$ heteroaryl or heteroar$C_{1-3}$ alkyl.

**26.** A compound as claimed in claim 25 wherein R is 2-thienylacetyl.

## Patentansprüche

**1.** Produkt zum Abgeben eines Arzneimittels an einen Wirt-Zielort, welches Produkt ein Immunkonjugat und ein Vor-Arzneimittel als eine kombinierte Präparation für sequentielle Verwendung in der Therapie umfaßt, welches Immunkonjugat in der Lage ist, ein oder mehrere Epitope, die mit dem Wirt-Zielort assoziiert sind, zu erkennen und an sie zu binden, und welches eine $\beta$-Lactamase-Wirkung besitzt, die das Vor-Arzneimittel am Zielort zum aktiven Arzneimittel oder einer instabilen Vorstufe davon hydrolysieren kann, dadurch gekennzeichnet, daß das Immunkonjugat ein $\beta$-Lactamaseenzym oder ein Fragment davon umfaßt, welche direkt mit einem Antikörper oder einem Antikörperfragment verbunden sind, die in der Lage sind, das eine oder die mehreren Epitope zu erkennen und an sie zu binden, und daß das Vor-Arzneimittel ein Cephalosporin- oder ein Penicillin-Derivat der Formel (3)

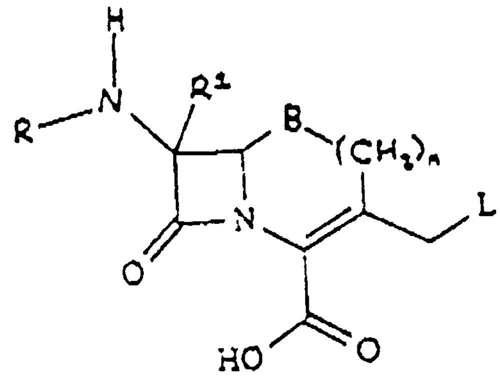

(3)

ist, worin R ein Acyl- oder ein Alkyl-Rest ist; $R^1$ ein Wasserstoffatom oder eine Alkoxygruppe ist; B $-CH_2-$, -O- oder -S- ist; n Null oder eine ganze Zahl von 1 bis einschließlich 4 ist; L das aktive Arzneimittel oder seine instabile Vorstufe ist, und L mit dem restlichen Teil des Moleküls derart verbunden ist, daß es eine Abspaltungsgruppe bildet, wodurch L bei Hydrolyse des Vor-Arzneimittels eliminiert wird.

**2.** Produkt nach Anspruch 1, worin L in der Formel (3) über ein in L vorhandenes Sauerstoff-, Stickstoff- oder Schwefelatom mit dem restlichen Teil des Moleküls verbunden ist.

**3.** Produkt nach Anspruch 1 oder 2, worin L in der Formel (3) eine Gruppe $-O-CO-L^1$ oder $-S-L^1$ ist, in welcher $L^1$ der restliche Teil des Arzneimittels oder seiner instabilen Vorstufe ist.

**4.** Produkt nach Anspruch 1, 2 oder 3, worin der Wirt-Zielort ein Tumor und L in der Formel (3) ein antineoplastisches Mittel oder eine instabile Vorstufe davon ist.

**5.** Produkt nach Anspruch 4, worin das antineoplastische Mittel ein Alkylierungsmittel, ein Antimetabolit, ein Antibiotikum, ein Mitoseinhibitor, ein Alkaloid, ein Hormon, ein Harnstoff, ein Hydrazin oder ein Imidazol ist.

**6.** Produkt nach Anspruch 5, worin das antineoplastische Mittel ein Alkylierungsmittel, ausgewählt aus Chlorambucil, Melphalan, Mechlorethamin, Cyclophosphamid, Uracilsenf, Triethylenphosphoramid, Triethylenthiophosphoramid, Busulphan oder Cisplatin, ist.

**7.** Produkt nach Anspruch 5, worin das antineoplastische Mittel ein Antimetabolit, ausgwählt aus Methotrexat, Fluoruracil und Floxuridin, Cytarabin, Mercaptopurin, Thioguanin, Fluoressigsäure oder Fluorzitronensäure, ist.

8. Produkt nach Anspruch 5, worin das antineoplastiche Mittel ein Antibiotikum, ausgewählt aus Bleomycinsulfat, Doxorubicin, Daunorubicin, Mitomycin C, Dactinomycin, Plicamycin, Calicheamicin oder Esperamicin, ist.

9. Produkt nach Anspruch 5, worin das antineoplastische Mittel ein Mitoseinhibitor, ausgewählt aus Etoposid, Vincristin oder Vinblastin, ist.

10. Produkt nach Anspruch 5, worin das antineoplastische Mittel das Alkaloid Ellipticin ist.

11. Produkt nach Anspruch 5, worin das antineoplastische Mittel ein Hormon, ausgewählt aus Dromostanolon, Testolacton, Megöstrolacetat, Medroxyprogesteronacetat, Diethylstilböstroldiphosphat, Polyöstradiolphosphat, Estramustinphosphat oder Tamoxiphen, ist.

12. Produkt nach Anspruch 5, worin das antineoplastische Mittel Hydroxyharnstoff ist.

13. Produkt nach Anspruch 5, worin das antineoplastische Mittel das Hydrazin Procarbazin ist.

14. Produkt nach Anspruch 5, worin das antineoplastiche Mittel das Imidazol Dacarbazin ist.

15. Produkt nach irgendeinem vorhergehenden Anspruch, worin das $\beta$-Lactamaseenzym oder sein aktives Fragment von Escherichia, Staphylokokken, Pseudomonas, Bacteriodes, Klebsiella, Citrobacter, Bacillus, Enterobacter oder Streptokokken ist.

16. Produkt nach Anspruch 15, worin das $\beta$-Lactamaseenzym oder sein aktives Fragment von B. cereus, Enterobacter cloacae oder E. coli ist.

17. Verbindung der Formel (3)

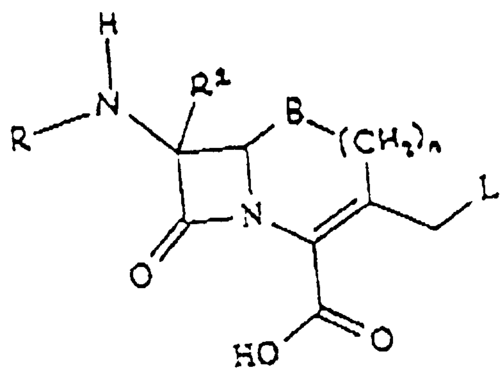

(3)

in der R ein Acyl- oder Alkyl-Rest ist; $R^1$ ein Wasserstoffatom oder eine Alkoxygruppe ist; B $-CH_2-$, -O- oder -S- ist; n Null oder eine ganze Zahl von 1 bis einschließlich 4 ist, und L mit dem restlichen Teil des Moleküls derart verbunden ist, daß es eine Abspaltungsgruppe bildet, dadurch gekennzeichnet, daß L ist: ein antineoplastisches Mittel, welches ein Alkylierungsmittel ist; ein Antimetabolit; ein Antibiotikum; ein Mitoseinhibitor; ein Alkaloid; ein Hormon; ein Harnstoff; ein Hydrazin; oder ein Imidazol, mit der Maßgabe, daß, wenn $R^1$ ein Wasserstoffatom ist, B -S- ist und $n=1$, L nicht ein Daunomycin, ein Adriamycin, ein Methotrexat, ein Mitomycin, ein Bleomycin, Melphalan, 6-Mercaptopurin, Cytosinarabinosid, ein Podophyllotoxin, ein Vinca-Arzneimittel, ein Difluornucleosid, eine Sulfonylharnstoff-Verbindung, ein Stickstoffsenf, ein Pteridin-Arzneimittel, ein Trichothecan, ein Colchicin oder 5-Fluoruracil ist.

18. Verbindung, wie in Anspruch 17 beansprucht, worin B -S- ist und n eine ganze Zahl 1 ist.

19. Verbindung, wie in Anspruch 17 oder 18 beansprucht, worin $R^1$ ein Wasserstoffatom ist.

20. Verbindung nach irgendeinem der Ansprüche 17-19, worin das antineoplastische Mittel L über ein in L vorhandenes Sauerstoff-, Stickstoff oder Schwefelatom an den Rest des Moleküls gebunden ist.

**21.** Verbindung, wie in Anspruch 20 beansprucht, worin das antineoplastische Mittel L -O-CO-$L^1$ ist, worin $L^1$ der restliche Teil des antineoplastischen Mittels oder dessen instabile Vorstufe ist.

**22.** Verbindung, wie in Anspruch 21 beansprucht, worin das antineoplastische Mittel L eine Gruppe -O-CO-$L^1$ ist und $L^1$ eine Gruppe -$NR^6R^7$ ist, worin $R^6$ und $R^7$ gleich oder verschieden sind und jede ein Wasserstoffatom oder eine gegebenenfalls substituierte $C_{1-6}$-Alkylgruppe ist, mit der Maßgabe, daß nur eine von $R^6$ und $R^7$ ein Wasserstoffatom ist; oder $L^1$ eine Gruppe -P-$NR^6R^7$ ist, wo P eine Phenylgruppe ist.

**23.** Verbindung, wie in Anspruch 21 beansprucht, worin L eine Thioguaningruppe ist.

**24.** Verbindung, wie in irgendeinem der Ansprüche 17-23 beansprucht, worin R ein gegebenenfalls substituierter aliphatischer, heteroaliphatischer, aromatischer, heteroaromatischer, araliphatischer oder heteroaraliphatischer carboxylischer oder Carbothiosäure-Rest oder ein Carbamoyl-Rest ist.

**25.** Verbindung, wie in Anspruch 24 beansprucht, worin R eine Gruppe $R^2C=X$ ist, wo X ein Sauerstoff- oder Schwefelatom ist und $R^2$ für ein Wasserstoffatom oder eine gegebenenfalls substituierte Gruppe steht, die aus Amino, substituiertem Amino, $C_{1-6}$-Alkyl, $C_{1-6}$-Alkylthio, $C_{6-12}$-Arylthio, $C_{1-6}$-Alkoxy, $C_{6-12}$-Aryloxy, $C_{2-6}$-Alkenyl oder -Alkynyl, Aryl, Ar$C_{1-3}$-Alkyl, $C_{3-6}$-Cycloalkyl, $C_{4-10}$-Heteroaryl oder Heteroar$C_{1-3}$-alkyl ausgewählt ist.

**26.** Verbindung, wie in Anspruch 25 beansprucht, worin R 2-Thienylacetyl ist.

**Revendications**

**1.** Produit pour délivrer un médicament vers un site cible chez un hôte, produit comprenant un immunoconjugué et un précurseur de médicament sous forme de préparation combinée destinée à agir séquentiellement en thérapeutique, ledit immunoconjugué étant capable de reconnaître et de se lier à un ou plusieurs épitopes associés au site cible chez l'hôte et doué d'activité $\beta$-lactamase ce qui le rend capable d'hydrolyser au niveau du site cible ledit précurseur de médicament en médicament actif ou en un précurseur instable de celui-ci, produit caractérisé en ce que ledit immunoconjugué comprend une enzyme $\beta$-lactamase ou un fragment de celle-ci directement lié à un anticorps ou à un fragment de ce dernier qui est capable de reconnaître et de se lier audit épitope ou auxdits épitopes, le précurseur de médicament étant un dérivé de la céphalosporine ou de la pénicilline de formule (3)

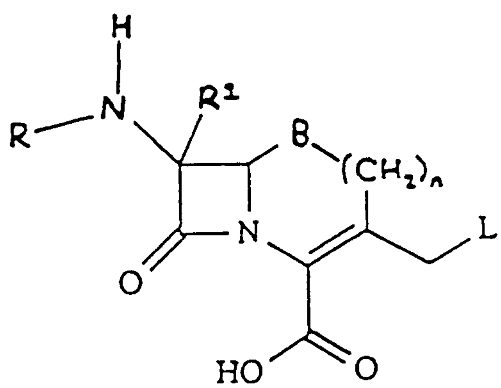

(3)

dans laquelle R est un radical acyle ou alkyle: $R^1$ est un atome d'hydrogène ou un groupe alcoxy; B est -$CH_2$, -O-, ou -S-, n est égal à zéro ou à un nombre entier variant de 1 à 4 inclus, L est un médicament actif ou un précurseur instable de celui-ci, L étant lié au reste de la molécule de sorte qu'elle forme un groupe partant afin de permettre l'élimination de L par hydrolyse du précurseur de médicament

**2.** Produit selon la revendication 1 dans lequel L dans la formule (3) est lié au reste de la molécule par l'intermédiaire d'un atome d'oxygène, d'azote ou de soufre présent dans L.

3. Produit selon la revendication 1 ou 2 dans lequel L dans la formule (3) est un groupe $-O-CO-L^1$ ou $-S-L^1$ où $L^1$ est la partie restante du médicament ou du précurseur instable de ce dernier.

4. Produit selon la revendication 1, 2 ou 3 dans lequel le site cible chez l'hôte est une tumeur, et L dans la formule (3) est un agent antinéoplastique ou un précurseur instable de ce dernier.

5. Produit selon la revendication 4 dans lequel l'agent antinéoplastique est un agent alkylant, un antimétabolite, un antibiotique, un inhibiteur de la mitose, un alcaloïde, une hormone, un dérivé de l'urée, ou un dérivé de l'hydrazine ou encore une imidazole.

6. Produit selon la revendication 5, dans lequel l'agent antinéoplastique est un agent alkylant choisi parmi le chlorambucil, le melphalan, le mechloréthamine, le cyclophosphamide, un analogue de l'uracile, le triéthylènephosphoramide, le triéthylènethiophosphoramide, le busulphan ou le cisplatine.

7. Produit selon la revendication 5, dans lequel l'agent antinéoplastique est un antimétabolite choisi parmi le méthotrexate, le fluorouracile et la floxuridine, la cytarabine, la mercaptopurine, la thioguanine, l'acide fluoroacétique ou l'acide fluorocitrique.

8. Produit selon la revendication 5, dans lequel l'agent antinéoplastique est un antibiotique choisi parmi le sulfate de bléomycine, la doxorubicine, la daunorubicine, la mitomycine C, la dactinomycine, la plicamycine, la calichéamycine, ou l'espéramycine.

9. Produit selon la revendication 5, dans lequel l'agent antinéoplastique est un inhibiteur de la mitose choisi parmi l'étoposide, la vincristine ou la vinblastine.

10. Produit selon la revendication 5, dans lequel l'agent antinéoplastique est l'alcaloïde ellipticine.

11. Produit selon la revendication 5, dans lequel l'agent antinéoplastique est une hormone choisie parmi la dromostanolone, la testolactone, l'acétate de mégestrol, l'acétate de médroxyprogestérone, le diphosphate de diéthylstilbestrol, le phosphate de polyestradiol, le phosphate d'estramustine ou le tamoxifen.

12. Produit selon la revendication 5, dans lequel l'agent antinéoplastique est l'hydroxyurée.

13. Produit selon la revendication 5, dans lequel l'agent antinéoplastique est l'hydrazine procarbazine.

14. Produit selon la revendication 5, dans lequel l'agent antinéoplastique est l'imidazole dacarbazine.

15. Produit selon l'une quelconque des revendications précédentes dans lequel l'enzyme $\beta$-lactamase ou un fragment actif de celle-ci provient de *Escherichia, Staphylococcus, Pseudomonas, Bactéroïdes, Klebsiella*, *Citrobacter, Bacillus, Enterobacter* ou de *Streptococcus.*

16. produit selon la revendication 15 dans lequel l'enzyme $\beta$-lactamase ou un fragment actif de celle-ci provient de *B. cereus, Enterobacter cloacae ou E. coli.*

**17.** Composé de formule (3):

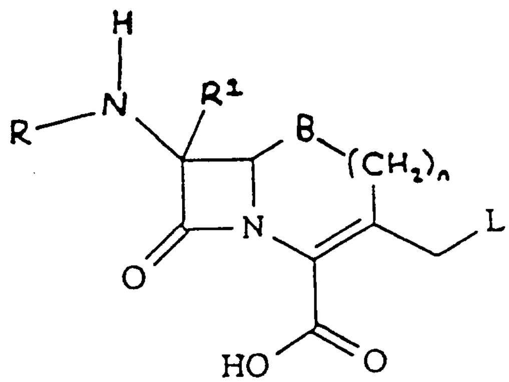

(3)

dans laquelle R est un radical acyle ou alkyle: $R^1$ est un atome d'hydrogène ou un groupe alcoxy; B est $-CH_2$, -O-, ou -S-, n est égal à zéro ou à un nombre entier variant de 1 à 4 inclus, L étant lié au reste de la molécule de sorte qu'il forme un groupe partant, composé caractérisé en ce que L est un agent antinéoplastique qui est un agent alkylant, un antimétabolite, un antibiotique, un inhibiteur de la mitose, un alcaloïde, une hormone, un dérivé de l'urée, ou un dérivé de l'hydrazine ou encore une imidazole pourvu que B soit -S- et n = 1 lorsque $R^1$ est un atome d'hydrogène, il s'en suit alors que L n'est ni une daunomycine, ni une adriamycine, ni un méthotrexate, ni une mitomycine, ni une bléomycine, ni le melphalan, ni la 6-mercaptopurine, ni la cytosine arabinoside, ni une podophyllotoxine, ni un médicament apparenté à la vincristine, ni un difluoronucléoside, un dérivé de la sulfonylurée, un dérivé de la moutarde azotée, ni un médicament apparenté à la ptéridine, ni un trichothécane, ni une colchicine ou la 5-fluorouracile.

**18.** Composé tel que revendiqué dans la revendication 17 dans lequel B est -S- et n est un nombre entier égal à 1.

**19.** Composé tel que revendiqué dans la revendication 17 ou 18 dans lequel $R^1$ est un atome d'hydrogène.

**20.** Composé selon l'une quelconque des revendications 17 à 19 dans lequel l'agent antinéoplastique L est lié au reste de la molécule par l'intermédiaire d'un atome d'oxygène, d'azote, ou de soufre présent dans L.

**21.** Composé tel que revendiqué la revendication 20 dans lequel l'agent antinéoplastique L est $-O-CO-L^1$ où $L^1$ est la partie restante de l'agent antinéoplastique ou d'un précurseur instable de ce dernier.

**22.** Composé tel que revendiqué dans la revendication 21 dans lequel l'agent antinéoplastique L est un groupe $-O-CO-L^1$ et $L^1$ est un groupe $-NR^6R^7$ dans lequel $R^6$ et $R^7$ sont identiques ou différents et représentent chacun un atome d'hydrogène ou un groupe alkyle en $C_{1-6}$ éventuellement substitué à condition que $R^6$ et $R^7$ soient l'un ou l'autre un atome d'hydrogène; ou $L^1$ est un groupe $-P-NR^6R^7$ où P est un groupe phényle.

**23.** Composé tel que revendiqué dans la revendication 21 dans lequel L est un groupe thioguanine.

**24.** Composé tel que revendiqué dans l'une quelconque des revendications 17 à 23 dans lequel R est un groupe aliphatique, hétéroaliphatique, aromatique, hétéroaromatique, araliphatique ou hétéroaraliphatique carboxylique éventuellement substitué ou un radical de type acide carbothioïque ou un groupe carbamoyle.

**25.** Composé tel que revendiqué dans la revendication 24 dans lequel R est un groupe $R^2C=X$ où X est un atome d'oxygène ou de soufre et $R^2$ représente un atome d'hydrogène ou un groupe éventuellement substitué choisi parmi le groupe amino éventuellement substitué, alkyle en $C_{1-6}$, alkylthio en $C_{1-6}$, arylthio en $C_{6-12}$, alcoxy en $C_{1-6}$, aryloxy en $C_{6-12}$, alcényle ou alkynyle en $C_{2-6}$, aryle, arakyle en $C_{1-3}$, cycloalkyle en $C_{3-6}$, hétéroaryle en $C_{4-10}$ ou hétéroaralkyle en $C_{1-3}$.

**26.** Composé tel que revendiqué dans la revendication 25 dans lequel R est un groupe 2-thiénylacétyle.